# EUROPÄISCHE PATENTSCHRIFT

(11) **EP 3 227 443 B1**
(45) Veröffentlichungstag und Bekanntmachung des Hinweises auf die Patenterteilung: **21.08.2019**
(21) Anmeldenummer: 15804382.8
(22) Anmeldetag: 01.12.2015
(51) Int. Cl.: C12N 9/54, C11D 3/386

(54) **PROTEASEVARIANTEN MIT VERBESSERTER WASCHLEISTUNG**
PROTEASE VARIANTS HAVING AN IMPROVED WASHING PERFORMANCE
VARIANTS DE PROTÉASES PRÉSENTANT UN MEILLEUR EFFET LAVANT

(30) Priorität: 04.12.2014 DE 102014224825
(43) Veröffentlichungstag der Anmeldung: 11.10.2017
(73) Patentinhaber: Henkel AG & Co. KGaA, 40589 Düsseldorf (DE)
(72) Erfinder: HELLMUTH, Hendrik, 40237 Düsseldorf (DE); WEBER, Thomas, 41541 Dormagen (DE); O'CONNELL, Timothy, 40547 Düsseldorf (DE); TONDERA, Susanne, 40597 Düsseldorf (DE); LAUFS, Brian, 41363 Jüchen (DE); AYDEMIR, Ayhan, 40589 Düsseldorf (DE); LINDNER, Claudia, 42719 Solingen (DE)
(86) Internationale Anmeldenummer: PCT/EP2015/078131
(87) Internationale Veröffentlichungsnummer: WO 2016/087403

(56) Entgegenhaltungen:
- WO-A1-2011/110625
- WO-A2-2012/080201
- DATABASE Geneseq [Online] 24. Oktober 2003 (2003-10-24), "Alkalische Protease aus Bacillus lentus (DSM 5483), Variante (S104T, S139Y, A224V).", XP002753509, gefunden im EBI accession no. GSP:AAR30127 Database accession no. AAR30127
- MAURER K-H ED - BETENBAUGH MICHAEL J ET AL: "Detergent proteases", CURRENT OPINION IN BIOTECHNOLOGY, LONDON, GB, Bd. 15, Nr. 4, 1. August 2004 (2004-08-01) , Seiten 330-334, XP002355089, ISSN: 0958-1669, DOI: 10.1016/J.COPBIO.2004.06.005
- BRYAN P N: "Protein engineering of subtilisin", BIOCHIMICA ET BIOPHYSICA ACTA. PROTEIN STRUCTURE AND MOLECULAR ENZYMOLOGY, ELSEVIER, AMSTERDAM; NL, Bd. 1543, Nr. 2, 29. Dezember 2000 (2000-12-29), Seiten 203-222, XP004279106, ISSN: 0167-4838, DOI: 10.1016/S0167-4838(00)00235-1

## Beschreibung

Die Erfindung liegt auf dem Gebiet der Enzymtechnologie. Die Erfindung betrifft Proteasen, deren Aminosäuresequenz insbesondere im Hinblick auf den Einsatz in Wasch- und Reinigungsmitteln verändert wurde, alle hinreichend ähnlichen Proteasen mit einer entsprechenden Veränderung und für sie codierende Nukleinsäuren. Die Erfindung betrifft ferner Verfahren und Verwendungen dieser Proteasen sowie sie enthaltende Mittel, insbesondere Wasch- und Reinigungsmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet beispielsweise der Artikel "Subtilases: Subtilisin-like Proteases" von R. Siezen, Seite 75-95 in "Subtilisin enzymes", herausgegeben von R. Bott und C. Betzel, New York, 1996. Subtilasen werden natürlicherweise von Mikroorganismen gebildet. Hierunter sind insbesondere die von Bacillus-Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die Protease aus Bacillus lentus, insbesondere aus Bacillus lentus DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so beispielsweise für den Einsatz in Wasch- und Reinigungsmitteln optimiert. Dazu gehören Punktmutagenese, Deletions- oder Insertionsmutagenese oder Fusion mit anderen Proteinen oder Proteinteilen. So sind für die meisten aus dem Stand der Technik bekannten Proteasen entsprechend optimierte Varianten bekannt. Aus WO 2012/080201 A2 sind Bacillus lentus DSM 5483 Subtilase Varianten bekannt und aus WO 2011/110625 A1 sind Bacillus gibsonii DSM 14391 Subtilase Varianten bekannt.

Überraschenderweise wurde gefunden, daß spezielle Varianten der alkalischen Protease aus Bacillus gibsonii (dsm 14391) [Seq. ID 1] oder hierzu hinreichend ähnliche Proteasen (bezogen auf die Sequenzidentität), besonders für den Einsatz in Wasch- oder Reinigungsmitteln geeignet und hinsichtlich der Waschleistung verbessert sind.

Gegenstand der vorliegenden Erfindung ist daher eine Protease, die eine Aminosäuresequenz umfasst, die zu der in SEQ ID NO. 1 angegebenen Aminosäuresequenz über deren Gesamtlänge zu mindestens 90% und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist und in der Zählung gemäß SEQ ID NO. 1 an Position 97 die Aminosäure D und/oder an Position 99 die Aminosäure E aufweist, wobei die Protease im Vergleich mit einer Protease gemäß SEQ ID NO. 1 eine verbesserte Reinigungsleistung aufweist.

Vorzugsweise weist die erfindungsgemäße Protease in der Zählung gemäß SEQ ID NO. 1 eine oder mehrere Aminosäuresubstitutionen auf, die ausgewählt sind unter N97D und R99E.

Besonders bevorzugte Proteasen sind solche, deren Aminosäurensequenzen mit den in Seq. ID 4 bis 6 angegebenen Aminosäuresequenzen identisch sind.

Ein weiterer Gegenstand der Erfindung ist ein Verfahren zur Herstellung einer Protease umfassend das Einbringen einer Aminosäuresubstitution N97D und R99E, in der Zählung gemäß SEQ ID NO. 1 in eine Ausgangsprotease, die zu der in SEQ ID NO. 1 angegebenen Aminosäuresequenz über deren Gesamtlänge zu mindestens 90% und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, wobei die Protease im Vergleich mit einer Protease gemäß SEQ ID NO. 1 eine verbesserte Reinigungsleistung aufweist.

Aminosäurepositionen, die im Rahmen der vorliegenden Erfindung mit der Formulierung "Zählung gemäß SEQ ID NO. 1" angegeben werden, verstehen sich folgendermaßen: Die weiteren Aminosäurepositionen werden durch ein Alignment der Aminosäuresequenz einer erfindungsgemäßen Protease mit der Aminosäuresequenz der Protease aus Bacillus gibsonii (dsm 14391), wie sie in SEQ ID NO. 1 angegeben ist, definiert. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere Zahl von Aminosäureresten umfasst als die Protease aus Bacillus gibsonii gemäß SEQ ID NO. 1. Ausgehend von den genannten Positionen in der Aminosäuresequenz der Protease aus Bacillus gibsonii sind die Veränderungspositionen in einer erfindungsgemäßen Protease diejenigen, die eben diesen Positionen in einem Alignement zugeordnet sind. Zum Beispiel weist die Protease gemäß SEQ ID NO.2 374 Aminosäuren auf. Im Alignement mit der Protease gemäß SEQ ID NO.1 ergibt sich daher, dass die Positionen 97 und 99 gemäß der Zählung nach SEQ ID NO.1 den Positionen 202 und 204 in der eigentlichen Aminosäuresequenz von SEQ ID NO.2 entsprechen. Eine Protease im Sinne der vorliegenden Patentanmeldung umfasst sowohl die Protease als solche als auch eine mit einem erfindungsgemäßen Verfahren hergestellte Protease. Alle Ausführungen zur Protease beziehen sich daher sowohl auf die Protease als Stoff wie auch auf die entsprechenden Verfahren, insbesondere Herstellungsverfahren der Protease.

Weitere Gegenstände der vorliegenden Erfindung sind für erfindungsgemäße Proteasen codierende Nukleinsäuren, erfindungsgemäße Proteasen oder Nukleinsäuren enthaltende nicht menschliche Wirtszellen sowie erfindungsgemäße Proteasen umfassende Mittel, insbesondere Wasch- und Reinigungsmittel, Wasch- und Reinigungsverfahren und Verwendungen der erfindungsgemäßen Proteasen, insbesondere in Wasch- und Reinigungsmitteln.

Eine erfindungsgemäße Veränderung N97D und/oder R99E in einer Protease, die eine zu der in SEQ ID NO. 1 angegebenen Aminosäuresequenz zu mindestens 90% identische Aminosäuresequenz umfasst, bewirkt eine verbesserte Reinigungsleistung dieser veränderten Protease in Wasch- und Reinigungsmitteln an mindestens einer protease-sensitiven Anschmutzung. Erfindungsgemäße Proteasen ermöglichen folglich eine verbesserte Entfernung von mindestens einer, bevorzugt von mehreren protease-sensitiven Anschmutzungen auf Textilien und/oder harten Oberflächen, beispielsweise Geschirr. Besonders vorteilhafte Reinigungsleistungen zeigen bevorzugte Ausführungsformen erfindungsgemäßer Proteasen an Blut-haltigen Anschmutzungen, beispielsweise an den Anschmutzungen
Blut-Milch/Tusche auf Baumwolle: Produkt Nr. C-05 erhältlich von CFT (Center For Testmaterials) B.V. Vlaardingen, Niederlande und
Vollei/Pigment auf Baumwolle: Produkt Nr. 10N erhältlich von wfk - Cleaning Technology Institute e.V., Krefeld, Deutschland.

Durch bevorzugte Ausführungsformen der vorliegenden Erfindung werden schmutzspezifische Proteasen bereitgestellt, deren Reinigungsleistung gezielt im Hinblick auf eine Anschmutzung oder auf mehrere Anschmutzungen ähnlichen Typs verbessert ist, insbesondere hinsichtlich Bluthaltiger und Vollei-haltiger Anschmutzungen.

Bevorzugte Ausführungsformen erfindungsgemäßer Proteasen erzielen solche vorteilhaften Reinigungsleistungen auch schon bei niedrigen Temperaturen, insbesondere in den

Temperaturbereichen zwischen 10°C und 60°C, bevorzugt zwischen 15°C und 50°C und besonders bevorzugt zwischen 20°C und 40°C. Weitere bevorzugte Ausführungsformen erfindungsgemäßer Proteasen erzielen solche vorteilhaften Reinigungsleistungen in einem weiten Temperaturbereich, beispielsweise zwischen 15°C und 90°C, vorzugsweise zwischen 20°C und 60°C.

Unter Reinigungsleistung wird im Rahmen der Erfindung die Aufhellungsleistung an einer oder mehreren Anschmutzungen, insbesondere auf Wäsche oder Geschirr, verstanden. Im Rahmen der Erfindung weist sowohl das Wasch- oder Reinigungsmittel, welches die Protease umfasst beziehungsweise die durch dieses Mittel gebildete Wasch- oder Reinigungsflotte, als auch die Protease selbst eine jeweilige Reinigungsleistung auf. Die Reinigungsleistung des Enzyms trägt somit zur Reinigungsleistung des Mittels beziehungsweise der durch das Mittel gebildeten Wasch- oder Reinigungsflotte bei. Die Reinigungsleistung wird bevorzugt ermittelt wie weiter unten angegeben.

Eine erfindungsgemäße Protease weist eine proteolytische Aktivität auf, das heißt, sie ist zur Hydrolyse von Peptidbindungen eines Polypeptids oder Proteins befähigt, insbesondere in einem Wasch- oder Reinigungsmittel. Eine erfindungsgemäße Protease ist daher ein Enzym, welches die Hydrolyse von Peptidbindungen katalysiert und dadurch in der Lage ist, Peptide oder Proteine zu spalten. Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife Enzyme.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. beispielsweise Altschul, S.F., Gish, W., Miller, W., Myers, E.W. & Lipman, D.J. (1990) "Basic local alignment search tool." J. Mol. Biol. 215:403-410, und Altschul, Stephan F., Thomas L. Madden, Alejandro A. Schaffer, Jinghui Zhang, Hheng Zhang, Webb Miller, and David J. Lipman (1997): "Gapped BLAST and PSI-BLAST: a new generation of protein database search programs"; Nucleic Acids Res., 25, S.3389-3402) und geschieht prinzipiell dadurch, daß ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. beispielsweise Chenna et al. (2003): Multiple sequence alignment with the Clustal series of programs. Nucleic Acid Research 31, 3497-3500), T-Coffee (vgl. beispielsweise Notredame et al. (2000): T-Coffee: A novel method for multiple sequence alignments. J. Mol. Biol. 302, 205-217) oder Programme, die auf diesen Programmen beziehungsweise Algorithmen basieren. In der vorliegenden Patentanmeldung wurden alle Sequenzvergleiche (Alignments) mit dem Computer-Programm Vector NTl® Suite 10.3 (Invitrogen Corporation, 1600 Faraday Avenue, Carlsbad, Kalifornien, USA) mit den vorgegebenen Standardparametern erstellt, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Die Reinigungsleistung einer erfindungsgemäßen Protease entspricht vorzugsweise mindestens derjenigen einer Protease, die eine Aminosäuresequenz umfasst, die der in SEQ ID NO. 1 angegebenen Aminosäuresequenz entspricht, und/oder mindestens derjenigen einer Protease, die eine Aminosäuresequenz umfasst, die der in SEQ ID NO. 3 angegebenen Aminosäuresequenz entspricht, wobei die Reinigungsleistung in einem Waschsystem bestimmt wird, das ein Waschmittel in einer Dosierung zwischen 4,5 und 7,0 Gramm pro Liter Waschflotte sowie die Protease enthält, wobei die zu vergleichenden Proteasen konzentrationsgleich (bezogen auf aktives Protein) eingesetzt sind und die Reinigungsleistung gegenüber einer Blut-Anschmutzung oder einer Vollei-Anschmutzung auf Baumwolle, insbesondere gegenüber der Anschmutzung Blut-Milch/Tusche auf Baumwolle: Produkt Nr. C-05 erhältlich von CFT (Center For Testmaterials) B.V. Vlaardingen, Niederlande oder
Vollei/Pigment auf Baumwolle: Produkt Nr. 10N erhältlich von wfk - Cleaning Technology Institute e.V., Krefeld, Deutschland, bestimmt wird durch Messung des Weißheitsgrades der gewaschenen Textilien, der Waschvorgang für 70 Minuten bei einer Temperatur von 40°C erfolgt und das Wasser eine Wasserhärte zwischen 15,5 und 16,5° (deutsche Härte) aufweist. Die Konzentration der Protease in dem für dieses Waschsystem bestimmten Waschmittel beträgt von 0,001-0,1 Gew.-%, vorzugsweise von 0,01 bis 0,06 Gew.-%, bezogen auf aktives Protein.

Ein bevorzugtes flüssiges Waschmittel für ein solches Waschsystem ist wie folgt zusammengesetzt (alle Angaben in Gewichts-Prozent): 0,3-0,5% Xanthan, 0,2-0,4% Anti-Schaummittel, 6-7% Glycerin, 0,3-0,5% Ethanol, 4-7% FAEOS (Fettalkoholethersulfat), 24-28% nichtionische Tenside, 1% Borsäure, 1-2% Natriumcitrat (Dihydrat), 2-4% Soda, 14-16% Kokosnuss-Fettsäuren, 0,5% HEDP (1-Hydroxyethan-(1,1-di-phosphonsäure)), 0-0,4% PVP (Polyvinylpyrrolidon), 0-0,05% optischer Aufheller, 0-0,001% Farbstoff, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Ein bevorzugtes pulverförmiges Waschmittel für ein solches Waschsystem ist wie folgt zusammengesetzt (alle Angaben in Gewichts-Prozent): 10% lineares Alkylbenzolsulfonat (Natrium-Salz), 1,5% C12-C18-Fettalkoholsulfat (Natrium-Salz), 2,0% C12-C18-Fettalkohol mit 7 EO, 20% Natriumcarbonat, 6,5% Natriumhydrogencarbonat, 4,0% amorphes Natriumdisilikat, 17% Natriumcarbonat-peroxohydrat, 4,0% TAED, 3,0% Polyacrylat, 1,0% Carboxymethylcellulose, 1,0% Phosphonat, 27% Natriumsulfat, Rest: Schauminhibitoren, optischer Aufheller, Duftstoffe. Bevorzugt beträgt die Dosierung des pulverförmigen Waschmittels zwischen 4,5 und 7,0 Gramm pro Liter Waschflotte, beispielsweise und besonders bevorzugt 4,7 Gramm pro Liter Waschflotte, oder 5,5, 5,9 oder 6,7 Gramm pro Liter Waschflotte. Bevorzugt wird gewaschen in einem pH-Wertebereich zwischen pH 9 und pH 11.

Die Bestimmung der Reinigungsleistung erfolgt vorzugsweise bei 40°C unter Verwendung eines flüssigen Waschmittels wie vorstehend angegeben, wobei der Waschvorgang vorzugsweise für 70 Minuten erfolgt.

Der Weißheitsgrad, d.h. die Aufhellung der Anschmutzungen, als Maß für die Reinigungsleistung wird bevorzugt mit optischen Messverfahren bestimmt, bevorzugt photometrisch. Ein hierfür geeignetes Gerät ist beispielsweise das Spektrometer Minolta CM508d. Üblicherweise werden die für die Messung eingesetzten Geräte zuvor mit einem Weißstandard, bevorzugt einem mitgelieferten Weißstandard, kalibriert.

Verfahren zur Bestimmung der Proteaseaktivität sind dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die ProteaseAktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20s bis 60s. Die Proteaseaktivität wird üblicherweise in Protease-Einheiten (PE) angegeben. Geeignete Proteaseaktivitäten betragen beispielsweise 2,25, 5 oder 10 PE pro ml Waschflotte. Die Proteaseaktivität ist jedoch nicht gleich Null.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration kann diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913) erfolgen.

Proteine können über die Reaktion mit einem Antiserum oder einem bestimmten Antikörper zu Gruppen immunologisch verwandter Proteine zusammengefasst werden. Die Angehörigen einer solchen Gruppe zeichnen sich dadurch aus, daß sie dieselbe, von einem Antikörper erkannte antigene Determinante aufweisen. Sie sind daher einander strukturell so ähnlich, daß sie von einem Antiserum oder bestimmten Antikörpern erkannt werden. Einen weiteren Erfindungsgegenstand bilden daher Proteasen, die dadurch gekennzeichnet sind, daß sie mindestens eine und zunehmend bevorzugt zwei, drei oder vier übereinstimmende antigene Determinanten mit einer erfindungsgemäßen Protease aufweisen. Solche Proteasen sind auf Grund ihrer immunologischen Übereinstimmungen den erfindungsgemäßen Proteasen strukturell so ähnlich, daß auch von einer gleichartigen Funktion auszugehen ist.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder-Deletionen, aufweisen. Solche Proteasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, zum Beispiel hinsichtlich ihrer Stabilität gegenüber Tensiden und/oder Bleichmitteln und/oder anderen Komponenten, kann daher im Rahmen der Erfindung zusätzlich weiterentwickelt sein.

Für die Beschreibung von Substitutionen, die genau eine Aminosäureposition betreffen (Aminosäureaustausche), wird folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere Austausche innerhalb derselben Polypeptidkette werden durch Schrägstriche voneinander getrennt. Bei Insertionen sind nach der Sequenzposition zusätzliche Aminosäuren benannt. Bei Deletionen ist die fehlende Aminosäure durch ein Symbol, beispielsweise einen Stern oder einen Strich, ersetzt. Beispielsweise beschreibt A95G die Substitution von Alanin an Position 95 durch Glycin, A95AG die Insertion von Glycin nach der Aminosäure Alanin an Position 95 und A95* die Deletion von Alanin an Position 95. Diese Nomenklatur ist dem Fachmann auf dem Gebiet der Enzymtechnologie bekannt.

Ein weiterer Gegenstand der Erfindung ist daher eine Protease, die in der Zählung gemäß SEQ ID NO. 1 an Position 97 die Aminosäure D und/oder an Position 99 die Aminosäure E aufweist und die aus einer erfindungsgemäßen Protease als Ausgangsmolekül durch ein- oder mehrfache konservative Aminosäuresubstitution erhältlich ist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T.

Ein weiterer Gegenstand der vorliegenden Erfindung ist eine Protease, die in der Zählung gemäß SEQ ID NO. 1 an Position 97 die Aminosäure D und/oder an Position 99 die Aminosäure E aufweist und die aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265 oder 266 zusammenhängenden Aminosäuren mit dem Ausgangsmolekül übereinstimmt.

So ist es beispielsweise möglich, an den Termini oder in den Loops des Enzyms einzelne Aminosäuren zu deletieren, ohne daß dadurch die proteolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms. Auch Substitutionen können vorteilhafte Wirkungen zeigen. Sowohl einzelne wie auch mehrere zusammenhängende Aminosäuren können gegen andere Aminosäuren ausgetauscht werden.

Alle genannten Sachverhalte sind auch auf die erfindungsgemäßen Verfahren zur Herstellung einer Protease anwendbar. Demnach umfasst ein erfindungsgemäßes Verfahren ferner einen oder mehreren der folgenden Verfahrensschritte:
(a) Einbringen einer ein- oder mehrfachen konservativen Aminosäuresubstitution in eine erfindungsgemäße Protease, wobei die Aminosäure D an Position 97 und/oder die Aminosäure E an Position 99 in der Zählung gemäß SEQ ID NO. 1 unverändert bleiben;
(b) Veränderung der Aminosäuresequenz einer Protease durch Fragmentierung, Deletions-, Insertions- oder Substitutionsmutagenese derart, daß die Protease eine Aminosäuresequenz umfasst, die über eine Länge von mindestens 50, 60, 70, 80, 90, 100, 110, 120, 130, 140, 150, 160, 170, 180, 190, 200, 210, 220, 230, 240, 250, 260, 265 oder 266 zusammenhängenden Aminosäuren mit dem Ausgangsmolekül übereinstimmt, wobei die Aminosäure D an Position 97 und/oder die Aminosäure E an Position 99 in der Zählung gemäß SEQ ID NO. 1 unverändert bleiben.

Sämtliche Ausführungen gelten auch für die erfindungsgemäßen Verfahren.

Eine erfindungsgemäße Protease kann zusätzlich stabilisiert sein, insbesondere durch eine oder mehrere Mutationen, beispielsweise Substitutionen, oder durch Kopplung an ein Polymer. Denn eine Erhöhung der Stabilität bei der Lagerung und/oder während des Einsatzes, beispielsweise beim Waschprozess, führt dazu, daß die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Muationen des Enzyms selbst erreicht werden, da solche Stabilisierungen im Anschluss an die Gewinnung des Enzyms keine weiteren Arbeitsschritte erfordern. Hierfür geeignete Sequenzveränderungen sind aus dem Stand der Technik bekannt. So können Proteasen beispielsweise dadurch stabilisiert werden, daß einer oder mehrere Tyrosin-Reste gegen andere Aminosäuren ausgetauscht werden.

Weitere Möglichkeiten der Stabilisierung sind z. B.:
- Veränderung der Bindung von Metallionen, insbesondere der Calcium-Bindungsstellen, beispielsweise durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Ein weiterer Gegenstand der Erfindung ist eine Protease wie vorstehend beschrieben, die dadurch gekennzeichnet ist, daß sie mindestens eine chemische Modifikation aufweist. Eine Protease mit einer solchen Veränderung wird als Derivat bezeichnet, d.h. die Protease ist derivatisiert.

Unter Derivaten werden im Sinne der vorliegenden Anmeldung demnach solche Proteine verstanden, deren reine Aminosäurekette chemisch modifiziert worden ist. Solche Derivatisierungen können beispielsweise in vivo durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch in vitro durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das beispielsweise über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können beispielsweise die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann beispielsweise für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit beispielsweise dessen Hautverträglichkeit zu erhöhen. Beispielsweise können Kopplungen mit makromolekularen Verbindungen, beispielsweise Polyethylenglykol, das Protein hinsichtlich der Stabilität und/oder Hautverträglichkeit verbessern.

Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen vergesellschaftet sein, beispielsweise aus der Kultur der produzierenden Mikroorganismen. Ein Protein kann auch, beispielsweise zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, daß es bei der Lagerung keine oder nur geringe Aktivität besitzt, und erst zum Zeitpunkt der Verwendung seine enzymatische Funktion entfaltet. Dies kann beispielsweise über entsprechende Begleitstoffe gesteuert werden. Insbesondere die gemeinsame Präparation von Proteasen mit Protease-Inhibitoren ist diesbezüglich möglich.

Betreffend alle vorstehend beschriebenen Proteasen beziehungsweise Proteasevarianten und/oder Derivate sind im Rahmen der vorliegenden Erfindung diejenigen besonders bevorzugt, deren Aktivität mindestens derjenigen der Protease gemäß SEQ ID NO. 1 und/oder SEQ ID NO. 3 entspricht, und/oder deren Reinigungsleistung mindestens derjenigen der Protease gemäß SEQ ID NO. 1 und/oder SEQ ID NO. 3 entspricht, wobei die Reinigungsleistung in einem Waschsystem bestimmt wird wie vorstehend beschrieben.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor.

Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, daß verschiedene Codons, also Basentriplets, für die gleichen Aminosäuren codieren können, so daß eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesen Erfindungsgegenstand mit eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, beispielsweise eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, daß in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie beispielsweise die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind beispielsweise aus Sambrook, J., Fritsch, E.F. and Maniatis, T. 2001. Molecular cloning: a laboratory manual, 3. Edition Cold Spring Laboratory Press. bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen beispielsweise solche, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren.

Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, beispielsweise durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, daß die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, beispielsweise durch Anknüpfung von Zuckermolekülen, Formylierungen, Aminierungen, usw. Solche posttranslationale Modifikationen können die Protease funktionell beeinflussen.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, daß sie die exprimierten Proteine nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so daß sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so daß sie über eine ähnliche Codon-Verwendung verfügen und ihr Protein-Syntheseapparat naturgemäß entsprechend ausgerichtet ist.

Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, daß sich durch den Einsatz solcher Mikroorganismen erfindungsgemäße Proteine herstellen lassen. Solche Mikroorganismen stellen dann Wirtszellen im Sinne der Erfindung dar.

In einer weiteren Ausführungsform der Erfindung ist die Wirtszelle dadurch gekennzeichnet, daß sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

Die Wirtszelle kann aber auch eine eukaryontische Zelle sein, die dadurch gekennzeichnet ist, daß sie einen Zellkern besitzt. Einen weiteren Gegenstand der Erfindung stellt daher eine Wirtszelle dar, die dadurch gekennzeichnet ist, daß sie einen Zellkern besitzt. Im Gegensatz zu prokaryontischen Zellen sind eukaryontische Zellen in der Lage, das gebildete Protein posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Proteins wünschenswert sein. Auch eine Coexpression mit den natürlicherweise von derartigen Zellen gebildeten Enzymen, wie beispielsweise Cellulasen oder Lipasen, kann vorteilhaft sein. Ferner können sich beispielsweise thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle
b) Isolieren der Protease aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise der erfindungsgemäßen Protease. Alle Fermentationsverfahren, die auf einem entsprechenden Verfahren zur Herstellung einer erfindungsgemäßen Protease beruhen, stellen Ausführungsformen dieses Erfindungsgegenstandes dar.

Fermentationsverfahren, die dadurch gekennzeichnet sind, daß die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse beziehungsweise Trockenmasse und/oder insbesondere in der Aktivität der interessierenden Protease erreicht werden. Ferner kann die Fermentation auch so gestaltet werden, daß unerwünschte Stoffwechselprodukte herausgefiltert oder durch Zugabe von Puffer oder jeweils passende Gegenionen neutralisiert werden.

Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, beispielsweise durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Proteasen herzustellen.

Ein weiterer Gegenstand der Erfindung ist ein Mittel, das dadurch gekennzeichnet ist, dass es eine erfindungsgemäße modifizierte Protease wie vorstehend beschrieben enthält. Bevorzugt ist das Mittel ein Wasch- oder Reinigungsmittel. Da erfindungsgemäße modifizierte Proteasen vorteilhafte Reinigungsleistungen insbesondere an Blut-haltigen und Vollei-haltigen Anschmutzungen aufweisen, sind die Mittel insbesondere zur Entfernung von solchen Anschmutzungen geeignet und vorteilhaft.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder bei der Handwäsche beziehungsweise -reinigung. Dazu gehören beispielsweise Waschmittel für Textilien, Teppiche, oder Naturfasern, für die die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln beispielsweise auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Der auf aktives Protein bezogene Gewichtsanteil der erfindungsgemäßen Protease am Gesamtgewicht erfindungsgemäßer Wasch- oder Reinigungsmittel beträgt vorzugsweise 0,005 bis 1,0 Gew.-%, bevorzugt 0,01 bis 0,5 Gew.-% und insbesondere 0,02 bis 0,2 Gew.-%. Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren (A. G. Gornall, C. S. Bardawill und M.M. David, J. Biol. Chem., 177 (1948), S. 751-766) bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al., J. Am. Chem. Soc. 88, 24 (1966), S. 5890-5913).

Die erfindungsgemäßen Wasch- oder Reinigungsmittel können weitere Enzyme enthalten. Als weitere Enzyme einsetzbar sind beispielsweise Lipasen oder Cutinasen, insbesondere wegen ihrer Triglycerid-spaltenden Aktivitäten, aber auch, um aus geeigneten Vorstufen in situ Persäuren zu erzeugen. Hierzu gehören beispielsweise die ursprünglich aus Humicola lanuginosa (Thermomyces lanuginosus) erhältlichen, beziehungsweise weiterentwickelten Lipasen, insbesondere solche mit dem Aminosäureaustausch D96L. Hierzu gehören beispielsweise die ursprünglich aus Humicola lanuginosa (Thermomyces lanuginosus) erhältlichen, beziehungsweise weiterentwickelten Lipasen, insbesondere solche mit einem oder mehreren der folgenden Aminosäureaustausche ausgehend von der genannten Lipase in den Positionen D96L, T213R und/oder N233R., besonders bevorzugt T213R und N233R. Des weiteren sind beispielsweise die Cutinasen einsetzbar, die ursprünglich aus Fusarium solani pisi und Humicola insolens isoliert worden sind. Einsetzbar sind weiterhin Lipasen, beziehungsweise Cutinasen, deren Ausgangsenzyme ursprünglich aus Pseudomonas mendocina und Fusarium solanii isoliert worden sind.

Die erfindungsgemäßen Mittel können auch Cellulasen oder Hemicellulasen wie Mannanasen, Xanthanlyasen, Pektinlyasen (=Pektinasen), Pektinesterasen, Pektatlyasen, Xyloglucanasen (=Xylanasen), Pullulanasen oder β-Glucanasen enthalten.

Zur Erhöhung der bleichenden Wirkung können erfindungsgemäß Oxidoreduktasen, beispielsweise Oxidasen, Oxygenasen, Katalasen, Peroxidasen, wie Halo-, Chloro-, Bromo-, Lignin-, Glucose- oder Mangan-peroxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

Als weitere Enzyme einsetzbar sind auch Amylasen. Für Amylasen können synonyme Begriffe verwendet werden, beispielsweise 1,4-alpha-D-Glucan-Glucanohydrolase oder Glycogenase. Erfindungsgemäß bevorzugte Amylasen sind α-Amylasen. Entscheidend dafür, ob ein Enzym eine α-Amylase im Sinne der Erfindung ist, ist deren Fähigkeit zur Hydrolyse von a(1-4)-Glykosidbindungen in der Amylose der Stärke.

Beispielhafte Amylasen sind die α-Amylasen aus Bacillus licheniformis, aus Bacillus amyloliquefaciens oder aus Bacillus stearothermophilus sowie insbesondere auch deren für den Einsatz in Wasch- oder Reinigungsmitteln verbesserte Weiterentwicklungen. Das Enzym aus Bacillus licheniformis ist von dem Unternehmen Novozymes unter dem Namen Termamyl® und von dem Unternehmen Danisco/Genencor unter dem Namen Purastar®ST erhältlich. Weiterentwicklungsprodukte dieser α-Amylase sind von dem Unternehmen Novozymes unter den Handelsnamen Duramyl® und Termamyl®ultra, von dem Unternehmen Danisco/Genencor unter dem Namen Purastar®OxAm und von dem Unternehmen Daiwa Seiko Inc., Tokyo, Japan, als Keistase® erhältlich. Die α-Amylase von Bacillus amyloliquefaciens wird von dem Unternehmen Novozymes unter dem Namen BAN® vertrieben, und abgeleitete Varianten von der α-Amylase aus Bacillus stearothermophilus unter den Namen BSG® und Novamyl®, ebenfalls von dem Unternehmen Novozymes. Desweiteren sind für diesen Zweck die α-Amylase aus Bacillus sp. A 7-7 (DSM 12368) und die Cyclodextrin-Glucanotransferase (CGTase) aus Bacillus agaradherens (DSM 9948) hervorzuheben. Ebenso sind Fusionsprodukte aller genannten Moleküle einsetzbar. Darüber hinaus sind die unter den Handelsnamen Fungamyl® von dem Unternehmen Novozymes erhältlichen Weiterentwicklungen der α-Amylase aus Aspergillus niger und A. oryzae geeignet. Weitere vorteilhaft einsetzbare Handelsprodukte sind beispielsweise die Amylase-LT® und Stainzyme® oder Stainzyme ultra® bzw. Stainzyme plus®, letztere ebenfalls von dem Unternehmen Novozymes. Auch durch Punktmutationen erhältliche Varianten dieser Enzyme können erfindungsgemäß eingesetzt werden. Besonders bevorzugte Amylasen sind offenbart in den internationalen Offenlegungsschriften WO 00/60060, WO 03/002711, WO 03/054177 und WO07/079938, auf deren Offenbarung daher ausdrücklich verwiesen wird bzw. deren diesbezüglicher Offenbarungsgehalt daher ausdrücklich in die vorliegende Patentanmeldung mit einbezogen wird.

Der auf aktives Protein bezogene Gewichtsanteil der weiteren Enzyme am Gesamtgewicht bevorzugter Wasch- oder Reinigungsmittels beträgt vorzugsweise 0,0005 bis 1,0 Gew.-%, bevorzugt 0,001 bis 0,5 Gew.-% und insbesondere 0,002 bis 0,2 Gew.-%.

Die Wasch- oder Reinigungsmittel können neben den zuvor beschriebenen Inhaltsstoffen reinigungsaktive Substanzen enthalten, wobei Substanzen aus der Gruppe der Tenside, Gerüststoffe, Polymere, Glaskorrosionsinhibitoren, Korrosionsinhibitoren, Duftstoffe und Parfümträger bevorzugt werden. Diese bevorzugten Inhaltsstoffe werden in der Folge näher beschrieben.

Ein bevorzugter Bestandteil der erfindungsgemäßen Wasch- oder Reinigungsmittel sind die nichtionischen Tenside, wobei nichtionische Tenside der allgemeinen Formel R¹-CH(OH)CH₂O-(AO)_{w}-(A'O)ₓ-(A"O)_{y}-(A"'O)_{z}-R², in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A, A', A" und A'" unabhängig voneinander für einen Rest aus der Gruppe -CH₂CH₂, -CH₂CH₂-CH₂, -CH₂-CH(CH₃), -CH₂-CH₂-CH₂-CH₂, -CH₂-CH(CH₃)-CH₂-, -CH₂-CH(CH₂-CH₃) stehen,
- w, x, y und z für Werte zwischen 0,5 und 120 stehen, wobei x, y und/oder z auch 0 sein können
bevorzugt sind.

Durch den Zusatz der vorgenannten nichtionischen Tenside der allgemeinen Formel R¹-CH(OH)CH₂O-(AO)_{w}-(A'O)ₓ-(A"O)_{y}-(A"'O)_{z}-R², nachfolgend auch als "Hydroxymischether" bezeichnet, kann die Reinigungsleistung erfindungsgemäßer Enzym-haltiger Zubereitungen deutlich verbessert werden und zwar sowohl im Vergleich zu Tensid-freien Systemen wie auch im Vergleich zu Systemen, die alternative nichtionischen Tenside, beispielsweise aus der Gruppe der polyalkoxylierten Fettalkohole enthalten.

Durch den Einsatz dieser nichtionischen Tenside mit einer oder mehreren freien Hydroxylgruppe an einem oder beiden endständigen Alkylreste kann die Stabilität der in den erfindungsgemäßen Wasch- oder Reinigungsmittelzubereitungen enthaltenen Enzyme deutlich verbessert werden.

Bevorzugt werden insbesondere solche endgruppenverschlossene poly(oxyalkylierten) Niotenside, die, gemäß der Formel R¹O[CH₂CH₂O]ₓCH₂CH(OH)R², neben einem Rest R¹, welcher für lineare oder verzweigte, gesättigte oder ungesättigte, aliphatische oder aromatische Kohlenwasserstoffreste mit 2 bis 30 Kohlenstoffatomen, vorzugsweise mit 4 bis 22 Kohlenstoffatomen steht, weiterhin einen linearen oder verzweigten, gesättigten oder ungesättigten, aliphatischen oder aromatischen Kohlenwasserstoffrest R² mit 1 bis 30 Kohlenstoffatomen aufweisen, wobei x für Werte zwischen 1 und 90, vorzugsweise für Werte zwischen 30 und 80 und insbesondere für Werte zwischen 30 und 60 steht.

Besonders bevorzugt sind Tenside der Formel R¹O[CH₂CH(CH₃)O]ₓ[CH₂CH₂O]_{y}CH₂CH(OH)R², in der R¹ für einen linearen oder verzweigten aliphatischen Kohlenwasserstoffrest mit 4 bis 18 Kohlenstoffatomen oder Mischungen hieraus steht, R² einen linearen oder verzweigten Kohlenwasserstoffrest mit 2 bis 26 Kohlenstoffatomen oder Mischungen hieraus bezeichnet und x für Werte zwischen 0,5 und 1,5 sowie y für einen Wert von mindestens 15 steht.
Zur Gruppe dieser nichtionischen Tenside zählen beispielsweise die C₂₋₂₆ Fettatkohot-(PO)₁-(EO)₁₅₋₄₀-2-hydroxyalkylether, insbesondere auch die C₈₋₁₀ Fettalkohol-(PO)₁-(EO)₂₂-2-hydroxydecylether.

Besonders bevorzugt werden weiterhin solche endgruppenverschlossene poly(oxyalkylierten) Niotenside der Formel R¹O[CH₂CH₂O]ₓ[CH₂CH(R³)O]_{y}CH₂CH(OH)R², in der R¹ und R² unabhängig voneinander für einen linearen oder verzweigten, gesättigten oder ein- bzw. mehrfach ungesättigten Kohlenwasserstoffrest mit 2 bis 26 Kohlenstoffatomen steht, R³ unabhängig voneinander ausgewählt ist aus -CH₃, -CH₂CH₃, -CH₂CH₂-CH₃, -CH(CH₃)₂, vorzugsweise jedoch für -CH₃ steht, und x und y unabhängig voneinander für Werte zwischen 1 und 32 stehen, wobei Niotenside mit R³ = -CH₃ und Werten für x von 15 bis 32 und y von 0,5 und 1,5 ganz besonders bevorzugt sind.

Weitere bevorzugt einsetzbare Niotenside sind die endgruppenverschlossenen poly(oxyalkylierten) Niotenside der Formel R¹O[CH₂CH(R³)O]ₓ[CH₂]ₖCH(OH)[CH₂]ⱼOR², in der R¹ und R² für lineare oder verzweigte, gesättigte oder ungesättigte, aliphatische oder aromatische Kohlenwasserstoffreste mit 1 bis 30 Kohlenstoffatomen stehen, R³ für H oder einen Methyl-, Ethyl-, n-Propyl-, iso-Propyl, n-Butyl-, 2-Butyl- oder 2-Methyl-2-Butylrest steht, x für Werte zwischen 1 und 30, k und j für Werte zwischen 1 und 12, vorzugsweise zwischen 1 und 5 stehen. Wenn der Wert x ≥ 2 ist, kann jedes R³ in der oben stehenden Formel R¹O[CH₂CH(R³)O]ₓ[CH₂]ₖCH(OH)[CH₂]ⱼOR² unterschiedlich sein. R¹ und R² sind vorzugsweise lineare oder verzweigte, gesättigte oder ungesättigte, aliphatische oder aromatische Kohlenwasserstoffreste mit 6 bis 22 Kohlenstoffatomen, wobei Reste mit 8 bis 18 C-Atomen besonders bevorzugt sind. Für den Rest R³ sind H, -CH₃ oder -CH₂CH₃ besonders bevorzugt. Besonders bevorzugte Werte für x liegen im Bereich von 1 bis 20, insbesondere von 6 bis 15.

Wie vorstehend beschrieben, kann jedes R³ in der oben stehenden Formel unterschiedlich sein, falls x ≥ 2 ist. Hierdurch kann die Alkylenoxideinheit in der eckigen Klammer variiert werden. Steht x beispielsweise für 3, kann der Rest R³ ausgewählt werden, um Ethylenoxid- (R³ = H) oder Propylenoxid- (R³ = CH₃) Einheiten zu bilden, die in jedweder Reihenfolge aneinandergefügt sein können, beispielsweise (EO)(PO)(EO), (EO)(EO)(PO), (EO)(EO)(EO), (PO)(EO)(PO), (PO)(PO)(EO) und (PO)(PO)(PO). Der Wert 3 für x ist hierbei beispielhaft gewählt worden und kann durchaus größer sein, wobei die Variationsbreite mit steigenden x-Werten zunimmt und beispielsweise eine große Anzahl (EO)-Gruppen, kombiniert mit einer geringen Anzahl (PO)-Gruppen einschließt, oder umgekehrt.

Besonders bevorzugte endgruppenverschlossene poly(oxyalkylierte) Alkohole der oben stehenden Formel weisen Werte von k = 1 und j = 1 auf, so dass sich die vorstehende Formel zu R¹O[CH₂CH(R³)O]ₓCH₂CH(OH)CH₂OR² vereinfacht. In der letztgenannten Formel sind R¹, R² und R³ wie oben definiert und x steht für Zahlen von 1 bis 30, vorzugsweise von 1 bis 20 und insbesondere von 6 bis 18. Besonders bevorzugt sind Tenside, bei denen die Reste R¹ und R² 9 bis 14 C-Atome aufweisen, R³ für H steht und x Werte von 6 bis 15 annimmt.

Als besonders wirkungsvoll haben sich schließlich die nichtionischen Tenside der allgemeine Formel R¹-CH(OH)CH₂O-(AO)_{w}-R² erwiesen, in der
- R¹ für einen geradkettigen oder verzweigten, gesättigten oder ein- bzw. mehrfach ungesättigten C₆₋₂₄-Alkyl- oder -Alkenylrest steht;
- R² für einen linearen oder verzweigten Kohlenwasserstoffrest mit 2 bis 26 Kohlenstoffatomen steht;
- A für einen Rest aus der Gruppe CH₂CH₂, -CH₂CH₂-CH₂, -CH₂-CH(CH₃) steht, und
- w für Werte zwischen 1 und 120, vorzugsweise 10 bis 80, insbesondere 20 bis 40 steht Zur Gruppe dieser nichtionischen Tenside zählen beispielsweise die C₄₋₂₂ Fettatkohot-(EO)₁₀₋₈₀-2-hydroxyalkylether, insbesondere auch die C₈₋₁₂ Fettalkohol-(EO)₂₂-2-hydroxydecylether und die C₄₋₂₂ Fettalkohol-(EO)₄₀₋₈₀-2-hydroxyalkylether

Bevorzugte Wasch- oder Reinigungsmittel sind dadurch gekennzeichnet, dass das Wasch- oder Reinigungsmittel mindestens ein nichtionisches Tensid, vorzugsweise ein nichtionisches Tensid aus der Gruppe der Hydroxymischether, enthält, wobei der Gewichtsanteil des nichtionischen Tensids am Gesamtgewicht des Wasch- oder Reinigungsmittels vorzugsweise 0,2 bis 10 Gew.-%, bevorzugt 0,4 bis 7,0 Gew.-% und insbesondere 0,6 bis 6,0 Gew.-% beträgt.

Bevorzugte erfindungsgemäße Wasch- oder Reinigungsmittel zur Verwendung in maschinellen Geschirrspülverfahren enthalten neben den zuvor beschriebenen nichtionischen Tensiden weitere Tenside, insbesondere amphotere Tenside enthalten. Der Anteil anionischer Tenside am Gesamtgewicht dieser Wasch- oder Reinigungsmittel ist jedoch vorzugsweise begrenzt. So sind bevorzugte maschinelle Geschirrspülmittel dadurch gekennzeichnet, dass diese bezogen auf ihr Gesamtgewicht weniger als 5, 0 Gew.-%, vorzugsweise weniger als 3,0 Gew.-%, besonders bevorzugt weniger als 2,0 Gew.-% Aniontensid enthalten. Auf den Einsatz von anionischen Tensiden in größerer Menge wird dabei insbesondere zur Vermeidung einer übermäßigen Schaumentwicklung verzichtet.

Ein weiterer bevorzugter Bestandteil erfindungsgemäßer Wasch- oder Reinigungsmittel sind Komplexbildner. Besonders bevorzugte Komplexbildner sind die Phosphonate. Die komplexbildenden Phosphonate umfassen neben der 1-Hydroxyethan-1,1-diphosphonsäure eine Reihe unterschiedlicher Verbindungen wie beispielsweise
Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z. B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Ein im Rahmen dieser Anmeldung bevorzugtes Wasch- oder Reinigungsmittel enthält ein oder mehrere Phosphonat(e) aus der Gruppe
a) Aminotrimethylenphosphonsäure (ATMP) und/oder deren Salze;
b) Ethylendiamintetra(methylenphosphonsäure) (EDTMP) und/oder deren Salze;
c) Diethylentriaminpenta(methylenphosphonsäure) (DTPMP) und/oder deren Salze;
d) 1-Hydroxyethan-1,1-diphosphonsäure (HEDP) und/oder deren Salze;
e) 2-Phosphonobutan-1,2,4-tricarbonsäure (PBTC) und/oder deren Salze;
f) Hexamethylendiamintetra(methylenphosphonsäure) (HDTMP) und/oder deren Salze;
g) Nitrilotri(methylenphosphonsäure) (NTMP) und/oder deren Salze.

Besonders bevorzugt werden Wasch- oder Reinigungsmittel, welche als Phosphonate 1-Hydroxyethan-1,1-diphosphonsäure (HEDP) oder Diethylentriaminpenta(methylenphosphonsäure) (DTPMP) enthalten. Selbstverständlich können die erfindungsgemäßen Wasch- oder Reinigungsmittel zwei oder mehr unterschiedliche Phosphonate enthalten. Bevorzugte erfindungsgemäße Wasch- oder Reinigungsmittel sind dadurch gekennzeichnet, dass das Wasch- oder Reinigungsmittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Wasch- oder Reinigungsmittels vorzugsweise 0,1 und 8,0 Gew.-%, bevorzugt 0,2 und 5,0 Gew.-% und insbesondere 0,5 und 3,0 Gew.-% beträgt.

Die erfindungsgemäßen Wasch- oder Reinigungsmittel enthalten weiterhin vorzugsweise Gerüststoff. Zu den Gerüststoffe zählen dabei insbesondere die Silikate, Aluminiumsilikaten (insbesondere Zeolithen), Salze organischer Di- und Polycarbonsäuren sowie Mischungen dieser Stoffe, vorzugsweise wasserlöslicher Buildersubstanzen Carbonate, organische Cobuilder, vorzugsweise wasserlöslicher Buildersubstanzen und -wo keine ökologischen Vorurteile gegen ihren Einsatz bestehen- auch die Phosphate.

Unter der Vielzahl der kommerziell erhältlichen Phosphate haben die Alkalimetallphosphate unter besonderer Bevorzugung von Pentanatriumtriphosphat, Na₅P₃O₁₀ (Natriumtripolyphosphat) bzw. Pentakaliumtriphosphat, K₅P₃O₁₀ (Kaliumtripolyphosphat) für die erfindungsgemäßen Mittel die größte Bedeutung. Werden im Rahmen der vorliegenden Anmeldung Phosphate als reinigungsaktive Substanzen in dem Wasch- oder Reinigungsmittel eingesetzt, so enthalten bevorzugte Mittel diese(s) Phosphat(e), vorzugsweise Pentakaliumtriphosphat, wobei der Gewichtsanteil des Phosphats am Gesamtgewicht des Wasch- oder Reinigungsmittels vorzugsweise 5,0 und 40 Gew.-%, bevorzugt 10 und 30 Gew.-% und insbesondere 12 und 25 Gew.-% beträgt.

In einer erfindungsgemäß besonders bevorzugten Ausführungsform wird auf den Einsatz von Phosphaten (auch Polyphosphaten) weitgehend oder vollständig verzichtet. Das Mittel enthält in dieser Ausführungsform vorzugsweise weniger als 5 Gew.-%, besonders bevorzugt weniger als 3 Gew.-%, insbesondere weniger als 1 Gew.-% Phosphat(e). Besonders bevorzugt ist das Mittel in dieser Ausführungsform völlig phosphatfrei, d.h. die Mittel enthalten weniger als 0,1 Gew.-% Phosphat(e).

Als organische Cobuilder sind insbesondere Polycarboxylate / Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben.

Erfindungsgemäß geeignete organische Gerüststoffe sind beispielsweise die in Form ihrer Natriumsalze einsetzbaren Polycarbonsäuren (Polycarboxylate), wobei unter Polycarbonsäuren solche Carbonsäuren verstanden werden, die mehr als eine, insbesondere zwei bis acht Säurefunktionen, bevorzugt zwei bis sechs, insbesondere zwei, drei, vier oder fünf Säurefunktionen im gesamten Molekül tragen. Bevorzugt sind als Polycarbonsäuren somit Dicarbonsäuren, Tricarbonsäuren Tetracarbonsäuren und Pentacarbonsäuren, insbesondere Di-, Tri- und Tetracarbonsäuren. Dabei können die Polycarbonsäuren noch weitere funktionelle Gruppen, wie beispielsweise Hydroxyl- oder Aminogruppen, tragen. Beispielsweise sind dies Citronensäure, Adipinsäure, Bernsteinsäure, Glutarsäure, Äpfelsäure, Weinsäure, Maleinsäure, Fumarsäure, Zuckersäuren (bevorzugt Aldarsäuren, beispielsweise Galactarsäure und Glucarsäure), Aminocarbonsäuren, insbesondere Aminodicarbonsäuren, Aminotricarbonsäuren, Aminotetracarbonsäuren wie beispielsweise Nitrilotriessigsäure (NTA), Glutamin-N,N-diessigsäure (auch als N,N-Bis(carboxymethyl)-L-glutaminsäure oder GLDA bezeichnet), Methylglycindiessigsäure (MGDA)) und deren Derivate sowie Mischungen aus diesen. Bevorzugte Salze sind die Salze der Polycarbonsäuren wie Citronensäure, Adipinsäure, Bernsteinsäure, Glutarsäure, Weinsäure, GLDA, MGDA und Mischungen aus diesen.
Weiterhin geeignet als organische Gerüststoffe sind polymere Polycarboxylate (organische Polymere mit einer Vielzahl, an (insbesondere größer zehn) Carboxylatfunktionen im Makromolekül), Polyaspartate, Polyacetale und Dextrine.
Bei den für polymere Polycarboxylate angegebenen Molmassen handelt es sich im Sinne dieser Schrift um gewichtsmittlere Molmassen M_{w} der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Schrift angegebenen Molmassen.

Geeignete Polymere sind insbesondere Polyacrylate, die bevorzugt eine Molekülmasse von 2000 bis 20000 g/mol aufweisen. Aufgrund ihrer überlegenen Löslichkeit können aus dieser Gruppe wiederum die kurzkettigen Polyacrylate, die Molmassen von 2000 bis 10000 g/mol, und besonders bevorzugt von 3000 bis 5000 g/mol, aufweisen, bevorzugt sein.

Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 2000 bis 70000 g/mol, vorzugsweise 20000 bis 50000 g/mol und insbesondere 30000 bis 40000 g/mol.

Auch Oxydisuccinate und andere Derivate von Disuccinaten, vorzugsweise Ethylendiamindisuccinat, sind weitere geeignete Cobuilder. Dabei wird Ethylendiamin-N,N'-disuccinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate.

Zur Verbesserung der Reinigungsleistung und/oder zur Einstellung der Viskosität enthalten bevorzugte Wasch- oder Reinigungsmittel mindestens ein hydrophob modifiziertes Polymer, vorzugsweise ein hydrophob modifziertes Carbonsäuregruppen-haltiges Polymer, wobei der Gewichtsanteil des hydrophob modifizierten Polymers am Gesamtgewicht des Wasch- oder Reinigungsmittels vorzugsweise 0,1 bis 10 Gew.-%, bevorzugt zwischen 0,2 und 8,0 Gew.-% und insbesondere 0,4 bis 6,0 Gew.-% beträgt.

In Ergänzung zu den zuvor beschriebenen Gerüststoffen können in dem Wasch- oder Reinigungsmittel reinigungsaktive Polymere enthalten sein. Der Gewichtsanteil der reinigungsaktiven Polymere am Gesamtgewicht erfindungsgemäßer maschineller Wasch- oder Reinigungsmittel beträgt vorzugsweise 0,1 bis 20 Gew.-%, vorzugsweise 1,0 bis 15 Gew.-% und insbesondere 2,0 bis 12 Gew.-%.

Als reinigungsaktive Polymere werden vorzugsweise Sulfonsäuregruppen-haltige Polymere, insbesondere aus der Gruppe der copolymeren Polysulfonate, eingesetzt. Diese copolymeren Polysulfonate enthalten neben Sulfonsäuregruppen-haltigem(n) Monomer(en) wenigstens ein Monomer aus der Gruppe der ungesättigten Carbonsäuren.

Als ungesättigte Carbonsäure(n) wird/werden mit besonderem Vorzug ungesättigte Carbonsäuren der Formel R¹(R²)C=C(R³)COOH eingesetzt, in der R¹ bis R³ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste wie vorstehend definiert oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist.

Besonders bevorzugte ungesättigte Carbonsäuren sind Acrylsäure, Methacrylsäure, Ethacrylsäure, α-Chloroacrylsäure, α-Cyanoacrylsäure, Crotonsäure, α-Phenyl-Acrylsäure, Maleinsäure, Maleinsäureanhydrid, Fumarsäure, Itaconsäure, Citraconsäure, Methylenmalonsäure, Sorbinsäure, Zimtsäure oder deren Mischungen. Einsetzbar sind selbstverständlich auch die ungesättigten Dicarbonsäuren.

Bei den Sulfonsäuregruppen-haltigen Monomeren sind solche der Formel
R⁵(R⁶)C=C(R⁷)-X-SO₃H bevorzugt, in der R⁵ bis R⁷ unabhängig voneinander für -H, -CH₃, einen geradkettigen oder verzweigten gesättigten Alkylrest mit 2 bis 12 Kohlenstoffatomen, einen geradkettigen oder verzweigten, ein- oder mehrfach ungesättigten Alkenylrest mit 2 bis 12 Kohlenstoffatomen, mit -NH₂, -OH oder -COOH substituierte Alkyl- oder Alkenylreste oder für -COOH oder -COOR⁴ steht, wobei R⁴ ein gesättigter oder ungesättigter, geradkettigter oder verzweigter Kohlenwasserstoffrest mit 1 bis 12 Kohlenstoffatomen ist, und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-, -C(O)-NH-C(CH₃)₂-CH₂- und -C(O)-NH-CH(CH₂CH₃)-.

Unter diesen Monomeren bevorzugt sind solche der Formeln H₂C=CH-X-SO₃H, H₂C=C(CH₃)-X-SO₃H und HO₃S-X-(R⁶)C=C(R⁷)-X-SO₃H, in denen R⁶ und R⁷ unabhängig voneinander ausgewählt sind aus -H, -CH₃, -CH₂CH₃, -CH₂CH₂CH₃, -CH(CH₃)₂ und X für eine optional vorhandene Spacergruppe steht, die ausgewählt ist aus -(CH₂)ₙ- mit n = 0 bis 4, -COO-(CH₂)ₖ- mit k = 1 bis 6, -C(O)-NH-C(CH₃)₂-,-C(O)-NH-C(CH₃)₂-CH₂- und -C(O)-NH-CH(CH₂CH₃)-.

Besonders bevorzugte Sulfonsäuregruppen-haltige Monomere sind dabei 1-Acrylamido-1-propansulfonsäure, 2-Acrylamido-2-propansulfonsäure, 2-Acrylamido-2-methyl-1-propansulfonsäure, 2-Methacrylamido-2-methyl-1-propansulfonsäure, 3-Methacrylamido-2-hydroxy-propansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen1-sulfonsäure, Styrolsulfonsäure, Vinylsulfonsäure, 3-Sulfopropylacrylat, 3-Sulfopropylmethacrylat, Sulfomethacrylamid, Sulfomethylmethacrylamid sowie Mischungen der genannten Säuren oder deren wasserlösliche Salze.

In den Polymeren können die Sulfonsäuregruppen ganz oder teilweise in neutralisierter Form vorliegen. Der Einsatz von teil- oder vollneutralisierten sulfonsäuregruppenhaltigen Copolymeren ist erfindungsgemäß bevorzugt.

Die Molmasse der erfindungsgemäß bevorzugt eingesetzten Sulfo-Copolymere kann variiert werden, um die Eigenschaften der Polymere dem gewünschten Verwendungszweck anzupassen. Bevorzugte maschinelle Geschirrspülmittel sind dadurch gekennzeichnet, dass die Copolymere Molmassen von 2000 bis 200.000 gmol⁻¹, vorzugsweise von 4000 bis 25.000 gmol⁻¹ und insbesondere von 5000 bis 15.000 gmol⁻¹ aufweisen.

In einer weiteren bevorzugten Ausführungsform umfassen die Copolymere neben Carboxylgruppen-haltigem Monomer und Sulfonsäuregruppen-haltigen Monomer weiterhin wenigstens ein nichtionisches, vorzugsweise hydrophobes Monomer. Durch den Einsatz dieser hydrophob modifizierten Polymere konnte insbesondere die Klarspülleistung erfindungsgemäßer maschineller Geschirrspülmittel verbessert werden.

Wasch- oder Reinigungsmittel, enthaltend ein Copolymer, umfassend
i) Carbonsäuregruppen-haltige Monomer(e)
ii) Sulfonsäuregruppen-haltige Monomer(e)
iii) nichtionische Monomer(e).

werden erfindungsgemäß bevorzugt. Durch den Einsatz dieser Terpolymere konnte die Klarspülleistung erfindungsgemäßer maschineller Geschirrspülmittel gegenüber vergleichbaren Geschirrspülmitteln, die Sulfopolymere ohne Zusatz nichtionischer Monomere enthalten, verbessert werden.

Als nichtionische Monomere werden vorzugsweise Monomere der allgemeinen Formel
R¹(R²)C=C(R³)-X-R⁴ eingesetzt, in der R¹ bis R³ unabhängig voneinander für -H, -CH₃ oder -C₂H₅ steht, X für eine optional vorhandene Spacergruppe steht, die ausgewählt ist aus -CH₂-, -C(O)O- und -C(O)-NH-, und R⁴ für einen geradkettigen oder verzweigten gesättigten Alkylrest mit 2 bis 22 Kohlenstoffatomen oder für einen ungesättigten, vorzugsweise aromatischen Rest mit 6 bis 22 Kohlenstoffatomen steht.

Besonders bevorzugte nichtionische Monomere sind Buten, Isobuten, Penten, 3-Methylbuten, 2-Methylbuten, Cyclopenten, Hexen, Hexen-1, 2-Methlypenten-1, 3-Methlypenten-1, Cyclohexen, Methylcyclopenten, Cyclohepten, Methylcyclohexen, 2,4,4-Trimethylpenten-1, 2,4,4-Trimethylpenten-2, 2,3-Dimethylhexen-1, 2,4-Diemthylhexen-1, 2,5-Dimethlyhexen-1, 3,5-Dimethylhexen-1, 4,4-Dimehtylhexan-1, Ethylcyclohexyn, 1-Octen, α-Olefine mit 10 oder mehr Kohlenstoffatomen wie beispielsweise 1-Decen, 1-Dodecen, 1-Hexadecen, 1-Oktadecen und C22-α-Olefin, 2-Styrol, α-Methylstyrol, 3-Methylstyrol, 4-Propylstryol, 4-Cyclohexylstyrol, 4-Dodecylstyrol, 2-Ethyl-4-Benzylstyrol, 1-Vinylnaphthalin, 2,Vinylnaphthalin, Acrylsäuremethylester, Acrylsäureethylester, Acrylsäurepropylester, Acrylsäurebutylester, Acrylsäurepentylester, Acrylsäurehexylester, Methacrylsäuremethylester, N-(Methyl)acrylamid, Acrylsäure-2-Ethylhexylester, Methacrylsäure-2-Ethylhexylester, *N*-(2-Ethylhexyl)acrylamid, Acrylsäureoctylester, Methacrylsäureoctylester, *N*-(Octyl)acrylamid, Acrylsäurelaurylester, Methacrylsäurelaurylester, *N*-(Lauryl)acrylamid, Acrylsäurestearylester, Methacrylsäurestearylester, *N*-(Stearyl)acrylamid, Acrylsäurebehenylester, Methacrylsäurebehenylester und *N*-(Behenyl)acrylamid oder deren Mischungen.

Der Gewichtsanteil der Sulfonsäuregruppen-haltigen Copolymere am Gesamtgewicht erfindungsgemäßer Wasch- oder Reinigungsmittel beträgt vorzugsweise 0,1 bis 15 Gew.-%, vorzugsweise 1,0 bis 12 Gew.-% und insbesondere 2,0 bis 10 Gew.-%.

Die erfindungsgemäßen Wasch- oder Reinigungsmittel können in den dem Fachmann bekannten Konfektionsformen, also beispielsweise in fester oder flüssiger Form aber auch als Kombination fester und flüssiger Angebotsformen vorliegen. Als feste Angebotsformen eignen sich insbesondere Pulver, Granulate, Extrudate oder Kompaktate, insbesondere Tabletten. Die flüssigen Angebotsformen auf Basis von Wasser und/oder organischen Lösungsmitteln können verdickt, in Form von Gelen vorliegen.

Die erfindungsgemäßen Wasch- oder Reinigungsmittel liegen vorzugsweise in flüssiger Form vor. Bevorzugte Wasch- oder Reinigungsmittel enthalten bezogen auf ihr Gesamtgewicht mehr als 40 Gew.-%, vorzugsweise zwischen 50 und 90 Gew.-% und insbesondere zwischen 60 und 80 Gew.- % Wasser.

Als einen weiteren Bestandteil können die erfindungsgemäßen Wasch- oder Reinigungsmittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Etheylenglykolmono-n-butylether, Diethylenglykolmethylether, Di-ethylenglykolethylether, Propylenglykolmethyl-, -ethyl- oder -propylether, Dipropylenglykolmethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether sowie Mischungen dieser Lösungsmittel. Der Gewichtsanteil dieser organischen Lösungsmittel am Gesamtgewicht erfindungsgemäßer Wasch- oder Reinigungsmittel beträgt vorzugsweise 0,1 bis 10 Gew.-%, bevorzugt 0,2 bis 8,0 Gew.-% und insbesondere 0,5 bis 5,0 Gew.-%. Ein besonders bevorzugtes und in Bezug auf die Stabilisierung der Wasch- oder Reinigungsmittel besonders wirksames organisches Lösungsmittel ist das Glycerin sowie das 1,2 Propylenglykol. Flüssige Wasch- oder Reinigungsmittel die mindestens ein Polyol, vorzugsweise aus der Gruppe Glycerin und 1,2-Propylenglycol enthalten, wobei der Gewichtsanteil des Polyols am Gesamtgewicht des Wasch- oder Reinigungsmittels vorzugsweise 0,1 und 10 Gew.-%, bevorzugt 0,2 und 8,0 Gew.-% und insbesondere 0,5 und 5,0 Gew.-% beträgt, werden erfindungsgemäß bevorzugt.
Weitere bevorzugte organische Lösungsmittel sind die organischen Amine und Alkanolamine. Die erfindungsgemäßen Wasch- oder Reinigungsmittel enthalten diese Amine vorzugsweise in Mengen von 0,1 bis 10 Gew.-%, bevorzugt von 0,2 bis 8,0 Gew.-% und insbesondere von 0,5 bis 5,0 Gew.-%, jeweils bezogen auf ihr Gesamtgewicht. Ein besonders bevorzugtes Alkanolamin ist das Ethanolamin.

Ein weiterer bevorzugter Bestandteil der erfindungsgemäßen Wasch- oder Reinigungsmittel ist ein Zuckeralkohol (Alditol). Die Gruppe der Alditole umfasst nichtcyclische Polyole der Formel HOCH₂[CH(OH)]ₙCH₂OH. Zu den Alditolen zählen beispielsweisee Mannit (Mannitol), Isomalt, Lactit, Sorbit (Sorbitol) und Xylit (Xylitol), Threit, Erythrit und Arabit. Als in Bezug auf die Enzymstabilität besonders vorteilhaft hat sich das Sorbitol erwiesen. Der Gewichtsanteil des Zuckeralkohols am Gesamtgewicht des Wasch- oder Reinigungsmittels beträgt vorzugsweise 1,0 bis 10 Gew.-%, bevorzugt 2,0 bis 8,0 Gew.-% und insbesondere 3,0 bis 6,0 Gew.-%. Erfindungsgemäße flüssige Wasch- oder Reinigungsmittel werden vorzugsweise in mehrphasiger Form, das heißt durch Kombination von zwei oder mehr voneinander getrennten unterschiedlichen flüssigen Wasch- oder Reinigungsmitteln konfektioniert. Diese Art der Konfektionierung erhöht die Stabilität des Wasch- oder Reinigungsmittels und verbessert dessen Reinigungsleistung. Ein erfindungsgemäß bevorzugtes Wasch- oder Reinigungsmittel ist dadurch gekennzeichnet, dass es ein Verpackungsmittel und zwei in diesem Verpackungsmittel befindlichen voneinander getrennte flüssige Wasch- oder Reinigungsmittel A und B umfasst, wobei die Zusammensetzung A
a) mindestens eine erfindungsgemäße modifizierte Protease;
b) mindestens ein weiteres, von der erfindungsgemäßen Protease verschiedenes Enzym,
c) 10 bis 84,9 Gew.-% Gerüststoff(e);
d) 15 bis 89,9 Gew.-% Wasser; enthält und
   die Zusammensetzung B
e) 10 bis 75 Gew.% Gerüststoff(e);
f) 25 bis 90 Gew.-% Wasser;
enthält.

Ein weiterer Erfindungsgegenstand ist ein Verfahren zur Reinigung von Textilien oder harten Oberflächen, das dadurch gekennzeichnet ist, daß in mindestens einem Verfahrensschritt ein erfindungsgemäßes Mittel angewendet wird, oder daß in mindestens einem Verfahrensschritt eine erfindungsgemäße Protease katalytisch aktiv wird, insbesondere derart, daß die Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

Hierunter fallen sowohl manuelle als auch maschinelle Verfahren, wobei maschinelle Verfahren bevorzugt sind. Verfahren zur Reinigung von Textilien zeichnen sich im allgemeinen dadurch aus, daß in mehreren Verfahrensschritten verschiedene reinigungsaktive Substanzen auf das Reinigungsgut aufgebracht und nach der Einwirkzeit abgewaschen werden, oder daß das Reinigungsgut in sonstiger Weise mit einem Waschmittel oder einer Lösung oder Verdünnung dieses Mittels behandelt wird. Entsprechendes gilt für Verfahren zur Reinigung von allen anderen Materialien als Textilien, insbesondere von harten Oberflächen. Alle denkbaren Wasch- oder Reinigungsverfahren können in wenigstens einem der Verfahrensschritte um die Anwendung eines erfindungsgemäßen Wasch- oder Reinigungsmittels oder einer erfindungsgemäßen Protease bereichert werden und stellen dann Ausführungsformen der vorliegenden Erfindung dar. Alle Sachverhalte, Gegenstände und Ausführungsformen, die für erfindungsgemäße Proteasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, daß diese Offenbarung auch für die vorstehenden erfindungsgemäßen Verfahren gilt.

Da erfindungsgemäße Proteasen natürlicherweise bereits eine hydrolytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, daß gewünschtenfalls als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Erfindungsgegenstand ist die Verwendung eines erfindungsgemäßen Mittels zur Reinigung von Textilien oder von harten Oberflächen, oder einer erfindungsgemäßen Protease zur Reinigung von Textilien oder von harten Oberflächen, insbesondere derart, daß die Protease in einer Menge von 40µg bis 4g, vorzugsweise von 50µg bis 3g, besonders bevorzugt von 100µg bis 2g und ganz besonders bevorzugt von 200µg bis 1g eingesetzt wird.

Alle Sachverhalte, Gegenstände und Ausführungsformen, die für erfindungsgemäße Proteasen und sie enthaltende Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, daß diese Offenbarung auch für die vorstehende erfindungsgemäße Verwendung gilt.

### Beispiele

Die folgenden Beispiele erläutern die Erfindung, ohne sie jedoch darauf einzuschränken:
Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laboratory Press, New York, 1989, oder vergleichbaren einschlägigen Werken angegeben sind. Enzyme und Baukästen (Kits) wurden nach den Angaben der jeweiligen Hersteller eingesetzt.

### Beispiel 1

Ausgehend von einer Protease, die eine Aminosäuresequenz gemäß SEQ ID NO. 1 aufwies, wurde eine erfindungsgemäße Protease-Variante durch ortsgerichtete ("site-directed") Mutagenese in der für die Protease codierenden Nukleinsäure hergestellt mittels "PHUSION Site-directed-Mutagenese-Kit" (Finnzyme, F541) hergestellt. Hierbei wurden die Codons für die angegebenen Aminosäurepositionen geändert, so daß bezogen auf die Aminosäuresequenz ein Austausch der Aminosäuren wie angegeben erfolgte. Die Expression der Protease-Variante erfolgte in fachüblicher Art- und Weise durch Transformation von Bacillus licheniformis, mit einem entsprechenden Expressionsvektor und nachfolgender Kultur der die Protease-Variante exprimierenden Transformanden.

Protease-Variante 1 [SEQ ID NO. 4]: Protease mit einer Aminosäuresequenz gemäß SEQ ID NO. 1 mit der Aminosäuresubstitution N97D in der Zählung gemäß SEQ ID NO. 1;

Protease-Variante 2 [SEQ ID NO. 5]: Protease mit einer Aminosäuresequenz gemäß SEQ ID NO. 1 mit der Aminosäuresubstitution R99E in der Zählung gemäß SEQ ID NO. 1;

Protease-Variante 3 [SEQ ID NO. 6]: Protease mit einer Aminosäuresequenz gemäß SEQ ID NO. 1 mit den Aminosäuresubstitutionen N97D und R99E in der Zählung gemäß SEQ ID NO. 1;

### Beispiel 2: Untersuchung der Varianten 1 bis 3 im Labormaßstab

Die Proteasevarianten 1 bis 3 [SEQ ID NO. 4, 5 und 6] wurden in Laborkleinfermentern nach Standardverfahren produziert.

Die Proteasen wurden mittels lonenaustauschchromatographie aus den
Fermentationsüberständen aufgereinigt und der Aktivproteingehalt der entstandenen Proben sowie die spezifische Aktivität mittels Titration der aktiven Zentren bestimmt. Mit dem Wildtyp [SEQ ID NO. 1] sowie der Referenzprotease [SEQ ID NO. 3] wurde entsprechend verfahren.

Ein erster Waschtest erfolgte im 48-Well-Maßstab auf Basis der ermittelten Daten aktivproteingleich bei 40°C in einer handelsähnlichen Screeningrezeptur gewaschen Folgende Ergebnisse wurden erzielt:

| | SEQ ID NO.3 | SEQ ID NO.1 | SEQ ID NO.4 | SEQ ID NO.5 | SEQ ID NO.6 |
|---|---|---|---|---|---|
| 10N | 4,2 | 6 | 7,7 | 5,9 | 8,3 |
| C-05 | 6,2 | 6,7 | 7,8 | 6,5 | 7,4 |

Es zeigt sich, dass die Mutation an der Position 97 zu einer verbesserten Waschleistung führt und teilweise die Kombination mit der Mutation an Position 99 eine weitere Verbesserung ermöglicht.

Für dieses Beispiel wurden standardisiert verschmutzte Textilien eingesetzt. Es wurden folgende Anschmutzungen verwendet:
- Blut-Milch/Tusche auf Baumwolle: Produkt Nr. C-05 erhältlich von CFT (Center For Testmaterials) B.V. Vlaardingen, Niederlande und
- Vollei/Pigment auf Baumwolle: Produkt Nr. 10N erhältlich von wfk - Cleaning Technology Institute e.V., Krefeld, Deutschland.

### Beispiel 3: Untersuchung der Varianten 1 bis 3 in der Waschmaschine

Die Proteasevarianten 1 bis 3 [SEQ ID NO. 4, 5 und 6] sowie der Wildtyp [SEQ ID NO. 1] wurden in der Waschmaschine vergleichend gewaschen.
Es wurde jeweils die gleiche Menge Aktivenzym eingesetzt.
Die Proteasen wurden on top zu einem wässrigen Flüssigwaschmittel (enthaltend neben Wasser 5,5 Gew.-% 7-fach ethoxylierten C12/14-Fettakohol, 5,3 Gew.-% Natrium-C9-13-Alkylbenzolsulfonat, 4,9 Gew.-% Natrium-C12/14-Fettalkoholethersulfat mit 2 EO, 1,8 Gew.-% Zitronensäure, 3 Gew.-% C12-18-Fettsäure, 0,1 Gew.-% Diethylentriaminpenta(methylenphosphonsäure)-hepta-Natriumsalz, 1,3 Gew.-% NaOH, 3,6 Gew.-% Ethanol/Glycerin) mit pH 8,5 ohne Protease dosiert und bei 40°C, Wasserhärte 16°dH, in einer Miele W 1935, Gesamtwaschzeit 2 h 15 min, gewaschen.
Es wurde eine 6-fach Bestimmung durchgeführt und es wurden verschiedene Anschmutzungsmonitore gewaschen und nach der Wäsche mit einem Farbmessgerät vermessen.

Es ergaben sich folgende delta Y-Werte im Vergleich zum Wildtyp [SEQ ID NO. 1]:

| | SEQ ID NO.5 | SEQ ID NO.4 | SEQ ID NO.6 |
|---|---|---|---|
| 01. EMPA 111 [CO] | 3,1 | 1,6 | 9,9 |
| 03. CFT C05 [CO] | 1,9 | 2,2 | 5,1 |
| 05. WFK 10EG [CO] | 0,2 | 0,7 | 1,2 |
| 09. H-MR-B [CO] | 7,4 | 4,2 | 8,4 |
| 10. EMPA 165 [CO] | 0,8 | 0,9 | 1,4 |

Auch für dieses Beispiel wurden standardisiert verschmutzte Textilien eingesetzt. Es wurden folgende Anschmutzungen verwendet:
- Blut auf Baumwolle; Produkt Nr. 111, erhältlich von der Firma Eidgenössische Material- und Prüfanstalt (EMPA) Testmaterialien AG, St. Gallen, Schweiz.
- Blut-Milch/Tusche auf Baumwolle: Produkt Nr. C-05 erhältlich von CFT (Center For Testmaterials) B.V. Vlaardingen, Niederlande und
- Eigelb auf Baumwolle: Produkt Nr. 10EG erhältlich von wfk - Cleaning Technology Institute e.V., Krefeld, Deutschland.
- Mit Standardschmutz angeschmutzte eingesetzte Testgewebe aus der Eigenfertigung der Anmelderin: Baumwolle, nicht ausgerüstet, verschmutzt mit Milch - Ruß (H-MR-B)
- Schokoladenpudding auf Baumwolle; Produkt Nr. 165, erhältlich von der Firma Eidgenössische Material- und Prüfanstalt (EMPA) Testmaterialien AG, St. Gallen, Schweiz.

Man erkennt deutlich, dass vor allem die Mutation R99E (in Variante 2, SEQ ID NO. 5) bzw. die Kombination R99E und N97D (in Variante 3, SEQ ID NO. 6) sehr vorteilhafte Waschleistungen liefern, im Vergleich zu ihrem Wildtyp [SEQ ID NO. 1].

### Beispiel 4: Untersuchung der Varianten 1 bis 3 (SEQ ID NO. 4, 5, 6) in der Geschirrspülmaschine

Es wurde ein Phosphat-freies automatisches Geschirrspülmittel in Form einer Geschirrspülmitteltablette verwendet. Das Tablettengewicht lag bei 19g. Die Geschirrspülmittelmatrix besaß die folgende Zusammensetzung:

**berechnet auf 20g Tablette**

| **Rohstoffe** | **Formelbereiche** | | | |
|---|---|---|---|---|
| | gesamt | | | |
| | % | | g/job | |
| Na-Citrat | 15,00 | 20,00 | 3,00 | 4,000 |
| Phosphonat (HEDP) | 2,50 | 7,50 | 0,50 | 1,500 |
| MGDA | 0,00 | 25,00 | 0,00 | 5,000 |
| Na-disilicat | 5,00 | 35,00 | 1,00 | 7,000 |
| Soda | 12,50 | 25,00 | 2,50 | 5,000 |
| Na-Percarbonat | 10,00 | 15,00 | 2,00 | 3,000 |
| Bleichkatalysator (Mn-basiert) | 0,02 | 0,50 | 0,003 | 0,100 |
| TAED | 2,00 | 3,00 | 0,40 | 0,600 |
| Nio Tensid 20-40EO end-cap mgl. | 2,50 | 10,00 | 0,50 | 2,000 |
| Polycarboxylat | 5,00 | 10,00 | 1,00 | 2,000 |
| kationisches Co-Polymer | 0,25 | 0,75 | 0,05 | 0,150 |
| crosslinked PVP - disintegrant | 0,00 | 1,50 | 0,00 | 0,300 |
| Protease | 1,50 | 5,00 | 0,30 | 1,000 |
| Amylase | 0,50 | 3,00 | 0,10 | 0,600 |
| Benzotriazol (Silberschutz) | 0,00 | 0,50 | 0,00 | 0,100 |
| Parfüm | 0,05 | 0,15 | 0,01 | 0,030 |
| Farbstofflösung | 0,00 | 1,00 | 0,00 | 0,200 |
| Zn-Acetat | 0,10 | 0,30 | 0,02 | 0,060 |
| Na-sulfat | 0,00 | 25,00 | 0,00 | 5,000 |
| Wasser | 0,00 | 1,50 | 0,00 | 0,300 |
| pH-Stellmittel (Citronensäure) | 1,00 | 1,50 | 0,20 | 0,300 |
| Prozesshilfsmittel | 0,00 | 5,00 | 0,00 | 1,000 |
| | 57,92 | 196,20 | 11,6 | 39,24 |

Die Reinigungsleistung beschreibt das Vermögen eines Geschirrspülmittels, insbesondere eines maschinellen Geschirrspülmittels, eine vorhandene Anschmutzung teilweise oder vollständig zu entfernen.

Es wurde die Reinigungsleistung des Mittels an drei verschiedenen Anschmutzungen getestet. Dem eingesetzten Mittel waren jeweils die erfindungsgemäßen Proteasevarianten gemäß SEQ ID NOs. 4, 5 oder 6, oder als Referenz die Protease mit der SEQ ID NO.1 zugesetzt.

Das Geschirrspülverfahren wurde in der Spülmaschine Miele GSL (Programm: 45°C, 8 min Haltezeit, Wasserhärte 21°deutscher Härte) nach IKW Standard durchgeführt. Die Geschirrspülmitteltablette wurde vor Beginn des Reinigungsprogramms in die Dosiervorrichtung gegeben.

Die Auswertung der Reinigungsleistung erfolgte visuell gemäß einer Skala von 1 bis 10, wobei der Wert 10 die beste Note ist (kein erkennbarer Rückstand). Es erfolgen 3 Wiederholungen mit jeweils 6 internen Replikaten pro Maschine. Die angegebenen Ergebnisse in Tabelle 1 und 2 sind der Mittelwert der Mehrfachbestimmung.

**Tabelle 1:**

| Proteasevariante | Assam-Tee | Tee BOP | Spaghetti |
|---|---|---|---|
| SEQ ID NO.1 | 2,3 | 3,3 | 3,8 |
| SEQ ID NO.4 | 3,0 | 4,0 | 3,9 |
| SEQ ID NO.5 | 2,7 | 4,4 | 3,5 |

Wie aus Tabelle 1 zu erkennen ist, führt die Verwendung der Proteasevarianten gemäß SEQ ID NO.4 und 5 zu einer Verbesserung der Reinigungsleistung, insbesondere auf Teeanschmutzungen.

**Tabelle 2:**

| Proteasevariante | Assam-Tee | Tee BOP | Spaghetti |
|---|---|---|---|
| SEQ ID NO.1 | 1,6 | 3,2 | 1,3 |
| SEQ ID NO.6 | 2,0 | 3,4 | 3,1 |

Wie aus Tabelle 2 zu erkennen ist, führt die Verwendung der Proteasevariante gemäß SEQ ID NO.6 sowohl auf Teeanschmutzungen, aber auch insbesondere auf Spaghettianschmutzungen zu einer Verbesserung der Reinigungsleistung.

## Patentansprüche

1. Protease, umfassend eine Aminosäuresequenz, die zu der in SEQ ID NO. 1 angegebenen Aminosäuresequenz über deren Gesamtlänge zu mindestens 90% und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist und in der Zählung gemäß SEQ ID NO. 1 an Position 97 die Aminosäure D und/oder an Position 99 die Aminosäure E aufweist, wobei die Protease im Vergleich mit einer Protease gemäß SEQ ID NO. 1 eine verbesserte Reinigungsleistung aufweist.

2. Protease nach Anspruch 1, **dadurch gekennzeichnet, dass** sie in der Zählung gemäß SEQ ID NO. 1 eine oder mehrere Aminosäuresubstitutionen aufweist, die ausgewählt sind aus N97D und R99E.

3. Protease nach einem der vorhergehenden Ansprüche, **dadurch gekennzeichnet, dass** ihre Aminosäuresequenz einer der in SEQ ID NO. 4 bis SEQ ID NO. 6 angegebenen Aminosäuresequenzen entspricht.

4. Verfahren zur Herstellung einer Protease umfassend das Einbringen einer Aminosäuresubstitution N97D und/oder R99E in der Zählung gemäß SEQ ID NO. 1 in eine Ausgangsprotease, die zu der in SEQ ID NO. 1 angegebenen Aminosäuresequenz über deren Gesamtlänge zu mindestens 90% und zunehmend bevorzugt zu mindestens 90,5%, 91%, 91,5%, 92%, 92,5%, 93%, 93,5%, 94%, 94,5%, 95%, 95,5%, 96%, 96,5%, 97%, 97,5%, 98%, 98,5% und 99% identisch ist, wobei die Protease im Vergleich mit einer Protease gemäß SEQ ID NO. 1 eine verbesserte Reinigungsleistung aufweist.

5. Nukleinsäure codierend für eine Protease nach einem der Ansprüche 1 bis 3 oder codierend für eine nach einem Verfahren des Anspruchs 4 erhältliche Protease.

6. Vektor enthaltend eine Nukleinsäure nach Anspruch 5, insbesondere ein Klonierungsvektor oder ein Expressionsvektor.

7. Nicht menschliche Wirtszelle, die eine Nukleinsäure nach Anspruch 5 oder einen Vektor nach Anspruch 6 beinhaltet, oder die eine Protease nach einem der Ansprüche 1 bis 3 beinhaltet, oder die eine nach einem Verfahren des Anspruchs 4 erhaltene Protease beinhaltet, insbesondere eine, die die Protease in das die Wirtszelle umgebende Medium sezerniert.

8. Verfahren zur Herstellung einer Protease umfassend
a) Kultivieren einer Wirtszelle gemäß Anspruch 7
b) Isolieren der Protease aus dem Kulturmedium oder aus der Wirtszelle.

9. Mittel, insbesondere ein Wasch- oder Reinigungsmittel, **dadurch gekennzeichnet, dass** es mindestens eine Protease nach einem der Ansprüche 1 bis 3 oder eine nach einem Verfahren des Anspruchs 4 erhältliche Protease enthält, insbesondere in einer Menge von 2µg bis 20mg pro g des Mittels.

## Claims

1. A protease comprising an amino acid sequence which is at least 90%, and, in order of increasing preference, at least 90.5%, 91%, 91.5%, 92%, 92.5%, 93%, 93.5%, 94%, 94.5%, 95%, 95.5%, 96%, 96.5%, 97%, 97.5%, 98%, 98.5% and 99%, identical to the amino acid sequence specified in SEQ ID NO. 1 over the entire length thereof and has, at position 97 in the numbering according to SEQ ID NO. 1, the amino acid D and/or, at position 99, the amino acid E, wherein the protease has improved cleaning performance in comparison with a protease according to SEQ ID NO. 1.

2. The protease according to claim 1, **characterized in that** it has, in the numbering according to SEQ ID NO. 1, one or more amino acid substitutions selected from N97D and R99E.

3. The protease according to one of the preceding claims, **characterized in that** its amino acid sequence corresponds to one of the amino acid sequences specified in SEQ ID NO. 4 to SEQ ID NO. 6.

4. A method for preparing a protease comprising introducing an amino acid substitution N97D and/or R99E in the numbering according to SEQ ID NO. 1 into a starting protease which is at least 90%, and, in order of increasing preference, at least 90.5%, 91%, 91.5%, 92%, 92.5%, 93%, 93.5%, 94%, 94.5%, 95%, 95.5%, 96%, 96.5%, 97%, 97.5%, 98%, 98.5% and 99%, identical to the amino acid sequence specified in SEQ ID NO. 1 over the entire length thereof, wherein the protease has improved cleaning performance in comparison with a protease according to SEQ ID NO. 1.

5. A nucleic acid that codes for a protease according to one of claims 1 to 3 or that codes for a protease that can be obtained by a method according to claim 4.

6. A vector containing a nucleic acid according to claim 5, in particular a cloning vector or an expression vector.

7. A non-human host cell that contains a nucleic acid according to claim 5 or a vector according to claim 6, or that contains a protease according to one of claims 1 to 3, or that contains a protease obtained by a method according to claim 4, in particular a non-human host cell that secretes the protease into the medium surrounding the host cell.

8. A method for preparing a protease, comprising
a) cultivation of a host cell according to claim 7
b) isolation of the protease from the culture medium or from the host cell.

9. An agent, in particular a washing or cleaning agent, **characterized in that** it contains at least one protease according to one of claims 1 to 3 or a protease that can be obtained by a method according to claim 4, in particular in an amount of from 2 µg to 20 mg per g of the agent.

## Revendications

1. Protéase comprenant une séquence d'acides aminés identique sur toute sa longueur à au moins 90 % et plus préférablement à au moins 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 % à la séquence d'acides aminés indiquée dans la séquence SEQ ID NO. 1 et présente, dans le comptage selon SEQ ID NO. 1, l'acide aminé D en position 97 et/ou l'acide aminé E en position 99, la protéase présentant, par rapport à une protéase selon SEQ ID NO. 1, un pouvoir nettoyant amélioré.

2. Protéase selon la revendication 1, **caractérisée en ce qu'**elle présente, dans le comptage selon SEQ ID NO. 1, au moins une substitution d'acides aminés choisie parmi N97D et R99E.

3. Protéase selon l'une des revendications précédentes, **caractérisée en ce que** sa séquence d'acides aminés correspond à l'une des séquences d'acides aminés indiquées dans SEQ ID NO. 4 à SEQ ID NO. 6.

4. Procédé de préparation d'une protéase, comprenant l'introduction d'une substitution d'acides aminés N97D et/ou R99E, dans le comptage selon SEQ ID NO. 1, dans une protéase de départ identique sur toute sa longueur à au moins 90 % et plus préférablement à au moins 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 % et 99 % à la séquence d'acides aminés indiquée dans SEQ ID NO. 1, la protéase présentant, par rapport à une protéase selon SEQ ID NO. 1, un pouvoir nettoyant amélioré.

5. Acide nucléique codant pour une protéase selon l'une des revendications 1 à 3 ou codant pour une protéase pouvant être obtenue par un procédé selon la revendication 4.

6. Vecteur contenant un acide nucléique selon la revendication 5, notamment un vecteur de clonage ou un vecteur d'expression.

7. Cellule hôte non humaine contenant un acide nucléique selon la revendication 5 ou un vecteur selon la revendication 6, ou comprenant une protéase selon l'une des revendications 1 ou 3, ou contenant une protéase obtenue par un procédé selon la revendication 4, notamment une protéase secrétant la protéase dans le milieu environnant la cellule hôte.

8. Procédé de préparation d'une protéase, consistant à
a) cultiver une cellule hôte selon la revendication 7,
b) isoler la protéase à partir du milieu de culture ou de la cellule hôte.

9. Agent, notamment détergent ou produit de nettoyage, **caractérisé en ce qu'**il contient au moins une protéase selon l'une des revendications 1 à 3 ou une protéase pouvant être obtenue par un procédé selon la revendication 4, notamment en une quantité comprise entre 2 µg et 20 mg par g de l'agent.


### SEQUENCE LISTING

<110> Henkel AG & Co. KGaA
<120> Proteasevarianten mit verbesserter Waschleistung
<130> PT032381
<150> 102014224825.6
   <151> 2014-12-04
<160> 9
<170> PatentIn version 3.5
<210> 1
   <211> 269
   <212> PRT
   <213> bacillus gibsonii (dsm 14391)
<400> 1
   Gln Gln Thr Val Pro Trp Gly Ile Thr Arg Val Gln Ala Pro Thr Val
   1               5                   10                  15
   His Asn Arg Gly Ile Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asn Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
           115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 2
   <211> 374
   <212> PRT
   <213> Bacillus sp. DSM 14392
<400> 2
   Met Gly Lys Ile Val Ala Gly Thr Ala Leu Ile Ile Ser Val Ala Phe
   1               5                   10                  15
   Ser Ser Ser Ile Ala Gln Ala Ala Glu Glu Ala Lys Glu Lys Tyr Leu
               20                  25                  30
   Ile Gly Phe Lys Glu Gln Glu Val Met Ser Gln Phe Val Asp Gln Ile
           35                  40                  45
   Asp Gly Asp Glu Tyr Ser Ile Ser Ser Gln Ala Glu Asp Val Glu Ile
       50                  55                  60
   Asp Leu Leu His Glu Phe Asp Phe Ile Pro Val Leu Ser Val Glu Leu
   65                  70                  75                  80
   Asp Pro Glu Asp Val Asp Ala Leu Glu Leu Asp Pro Ala Ile Ala Tyr
                   85                  90                  95
   Ile Glu Glu Asp Ala Glu Val Thr Thr Met Gln Thr Val Pro Trp Gly
               100                 105                 110
   Ile Asn Arg Val Gln Ala Pro Ile Ala Gln Ser Arg Gly Phe Thr Gly
           115                 120                 125
   Thr Gly Val Arg Val Ala Val Leu Asp Thr Gly Ile Ser Asn His Ala
       130                 135                 140
   Asp Leu Arg Ile Arg Gly Gly Ala Ser Phe Val Pro Gly Glu Pro Asn
   145                 150                 155                 160
   Ile Ser Asp Gly Asn Gly His Gly Thr His Val Ala Gly Thr Ile Ala
                   165                 170                 175
   Ala Leu Asn Asn Ser Ile Gly Val Leu Gly Val Ala Pro Asn Val Asp
               180                 185                 190
   Leu Tyr Gly Val Lys Val Leu Gly Ala Ser Gly Ser Gly Ser Ile Ser
           195                 200                 205
   Gly Ile Ala Gln Gly Leu Gln Trp Ala Ala Asn Asn Gly Met His Ile
       210                 215                 220
   Ala Asn Met Ser Leu Gly Ser Ser Ala Gly Ser Ala Thr Met Glu Gln
   225                 230                 235                 240
   Ala Val Asn Gln Ala Thr Ala Ser Gly Val Leu Val Val Ala Ala Ser
                   245                 250                 255
   Gly Asn Ser Gly Ala Gly Asn Val Gly Phe Pro Ala Arg Tyr Ala Asn
               260                 265                 270
   Ala Met Ala Val Gly Ala Thr Asp Gln Asn Asn Asn Arg Ala Ser Phe
           275                 280                 285
   Ser Gln Tyr Gly Ala Gly Leu Asp Ile Val Ala Pro Gly Val Gly Val
       290                 295                 300
   Gln Ser Thr Val Pro Gly Asn Gly Tyr Ala Ser Phe Asn Gly Thr Ser
   305                 310                 315                 320
   Met Ala Thr Pro His Val Ala Gly Val Ala Ala Leu Val Lys Gln Lys
                   325                 330                 335
   Asn Pro Ser Trp Ser Asn Val Gln Ile Arg Asn His Leu Lys Asn Thr
               340                 345                 350
   Ala Thr Asn Leu Gly Asn Thr Thr Gln Phe Gly Ser Gly Leu Val Asn
           355                 360                 365
   Ala Glu Ala Ala Thr Arg
   370
<210> 3
   <211> 269
   <212> PRT
   <213> Bacillus lentus
<400> 3
   Ala Gln Ser Val Pro Trp Gly Ile Ser Arg Val Gln Ala Pro Ala Ala
   1               5                   10                  15
   His Asn Arg Gly Leu Thr Gly Ser Gly Val Lys Val Ala Val Leu Asp
               20                  25                  30
   Thr Gly Ile Ser Thr His Pro Asp Leu Asn Ile Arg Gly Gly Ala Ser
           35                  40                  45
   Phe Val Pro Gly Glu Pro Ser Thr Gln Asp Gly Asn Gly His Gly Thr
       50                  55                  60
   His Val Ala Gly Thr Ile Ala Ala Leu Asn Asn Ser Ile Gly Val Leu
   65                  70                  75                  80
   Gly Val Ala Pro Ser Ala Glu Leu Tyr Ala Val Lys Val Leu Gly Ala
                   85                  90                  95
   Asp Gly Glu Gly Ala Ile Ser Ser Ile Ala Gln Gly Leu Glu Trp Ala
               100                 105                 110
   Gly Asn Asn Gly Met His Val Ala Asn Leu Ser Leu Gly Ser Pro Ser
           115                 120                 125
   Pro Ser Ala Thr Leu Glu Gln Ala Val Asn Ser Ala Thr Ser Arg Gly
       130                 135                 140
   Val Leu Val Val Ala Ala Ser Gly Asn Ser Gly Ala Ser Ser Ile Ser
   145                 150                 155                 160
   Tyr Pro Ala Arg Tyr Ala Asn Ala Met Ala Val Gly Ala Thr Asp Gln
                   165                 170                 175
   Asn Asn Asn Arg Ala Ser Phe Ser Gln Tyr Gly Ala Gly Leu Asp Ile
               180                 185                 190
   Val Ala Pro Gly Val Asn Val Gln Ser Thr Tyr Pro Gly Ser Thr Tyr
           195                 200                 205
   Ala Ser Leu Asn Gly Thr Ser Met Ala Thr Pro His Val Ala Gly Ala
       210                 215                 220
   Ala Ala Leu Val Lys Gln Lys Asn Pro Ser Trp Ser Asn Val Gln Ile
   225                 230                 235                 240
   Arg Asn His Leu Lys Asn Thr Ala Thr Ser Leu Gly Ser Thr Asn Leu
                   245                 250                 255
   Tyr Gly Ser Gly Leu Val Asn Ala Glu Ala Ala Thr Arg
               260                 265
<210> 4
   <211> 269
   <212> PRT
   <213> bacillus gibsonii (dsm 14391)
<400> 4
   Gln Gln Thr Val Pro Trp Gly Ile Thr Arg Val Gln Ala Pro Thr Val
   1               5                   10                  15
   His Asn Arg Gly Ile Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65                  70                  75                  80
   Gly Val Ala Pro Ser Ala Asp Leu Tyr Ala Val Lys Val Leu Gly Ala
                   85                  90                  95
   Asp Gly Arg Gly Ser Val Ser Gly Ile Ala Gln Gly Leu Glu Trp Ala
               100                 105                 110
   Ala Thr Asn Asn Met His Ile Ala Asn Met Ser Leu Gly Ser Asp Ala
           115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
   165 170 175
   Asn Asn Arg Arg Ala Ser Phe Ser Gln Tyr Gly Thr Gly Ile Asp Ile
   180 185 190
   Val Ala Pro Gly Val Gly Ile Gln Ser Thr Tyr Leu Asn Asn Ser Tyr
   195 200 205
   Ala Ser Met Pro Gly Thr Ser Met Ala Thr Pro His Val Ala Gly Val
   210 215 220
   Ala Ala Leu Val Lys Gln Lys Asn Pro Ser Trp Asn Ala Thr Gln Ile
   225 230 235 240
   Arg Asn His Leu Lys Asn Thr Ala Thr Asn Leu Gly Asn Ser Ser Gln
   245 250 255
   Phe Gly Ser Gly Leu Val Asn Ala Asp Ala Ala Thr Arg
   260 265
<210> 5
   <211> 269
   <212> PRT
   <213> bacillus gibsonii (dsm 14391)
<400> 5
   Gln Gln Thr Val Pro Trp Gly Ile Thr Arg Val Gln Ala Pro Thr Val
   1 5 10 15
   His Asn Arg Gly Ile Thr Gly Ser Gly Val Lys Val Ala Ile Leu Asp
   20 25 30
   Thr Gly Ile Ala Gln His Ser Asp Leu Thr Ile Arg Gly Gly Ala Ser
   35 40 45
   Phe Val Pro Gly Glu Ser Thr Thr Ala Asp Leu Asn Gly His Gly Thr
   50 55 60
   His Val Ala Gly Thr Val Ala Ala Leu Asn Asn Ser Ile Gly Val Ile
   65 70 75 80
   Gly Val Ala Pro Ser Ala Asp Leu Tyr Ala Val Lys Val Leu Gly Ala
   85 90 95
   Asn Gly Glu Gly Ser Val Ser Gly Ile Ala Gln Gly Leu Glu Trp Ala
   100 105 110
   Ala Thr Asn Asn Met His Ile Ala Asn Met Ser Leu Gly Ser Asp Ala
           115                 120                 125
   Pro Ser Thr Thr Leu Glu Arg Ala Val Asn Tyr Ala Thr Ser Arg Gly
       130                 135                 140
   Val Leu Val Ile Ala Ala Thr Gly Asn Asn Gly Thr Gly Ser Ile Gly
   145                 150                 155                 160
   Tyr Pro Ala Arg Tyr Ala Asn Ala Met Ala Val Gly Ala Thr Asp Gln
                   165                 170                 175
   Asn Asn Arg Arg Ala Ser Phe Ser Gln Tyr Gly Thr Gly Ile Asp Ile
               180                 185                 190
   Val Ala Pro Gly Val Gly Ile Gln Ser Thr Tyr Leu Asn Asn Ser Tyr
           195                 200                 205
   Ala Ser Met Pro Gly Thr Ser Met Ala Thr Pro His Val Ala Gly Val
       210                 215                 220
   Ala Ala Leu Val Lys Gln Lys Asn Pro Ser Trp Asn Ala Thr Gln Ile
   225                 230                 235                 240
   Arg Asn His Leu Lys Asn Thr Ala Thr Asn Leu Gly Asn Ser Ser Gln
                   245                 250                 255
   Phe Gly Ser Gly Leu Val Asn Ala Asp Ala Ala Thr Arg
               260                 265
<210> 6
   <211> 269
   <212> PRT
   <213> bacillus gibsonii (dsm 14391)
<400> 6
   Gln Gln Thr Val Pro Trp Gly Ile Thr Arg Val Gln Ala Pro Thr Val
   1               5                   10                  15
   His Asn Arg Gly Ile Thr Gly Ser Gly Val Lys Val Ala Ile Leu Asp
               20                  25                  30
   Thr Gly Ile Ala Gln His Ser Asp Leu Thr Ile Arg Gly Gly Ala Ser
           35                  40                  45
   Phe Val Pro Gly Glu Ser Thr Thr Ala Asp Leu Asn Gly His Gly Thr
       50                  55                  60
   His Val Ala Gly Thr Val Ala Ala Leu Asn Asn Ser Ile Gly Val Ile
   65 70 75 80
   Gly Val Ala Pro Ser Ala Asp Leu Tyr Ala Val Lys Val Leu Gly Ala
   85 90 95
   Asp Gly Glu Gly Ser Val Ser Gly Ile Ala Gln Gly Leu Glu Trp Ala
   100 105 110
   Ala Thr Asn Asn Met His Ile Ala Asn Met Ser Leu Gly Ser Asp Ala
   115 120 125
   Pro Ser Thr Thr Leu Glu Arg Ala Val Asn Tyr Ala Thr Ser Arg Gly
   130 135 140
   Val Leu Val Ile Ala Ala Thr Gly Asn Asn Gly Thr Gly Ser Ile Gly
   145 150 155 160
   Tyr Pro Ala Arg Tyr Ala Asn Ala Met Ala Val Gly Ala Thr Asp Gln
   165 170 175
   Asn Asn Arg Arg Ala Ser Phe Ser Gln Tyr Gly Thr Gly Ile Asp Ile
   180 185 190
   Val Ala Pro Gly Val Gly Ile Gln Ser Thr Tyr Leu Asn Asn Ser Tyr
   195 200 205
   Ala Ser Met Pro Gly Thr Ser Met Ala Thr Pro His Val Ala Gly Val
   210 215 220
   Ala Ala Leu Val Lys Gln Lys Asn Pro Ser Trp Asn Ala Thr Gln Ile
   225 230 235 240
   Arg Asn His Leu Lys Asn Thr Ala Thr Asn Leu Gly Asn Ser Ser Gln
   245 250 255
   Phe Gly Ser Gly Leu Val Asn Ala Asp Ala Ala Thr Arg
   260 265
<210> 7
   <211> 374
   <212> PRT
   <213> Bacillus sp. DSM 14392
<400> 7
   Met Gly Lys Ile Val Ala Gly Thr Ala Leu Ile Ile Ser Val Ala Phe
   1 5 10 15
   Ser Ser Ser Ile Ala Gln Ala Ala Glu Glu Ala Lys Glu Lys Tyr Leu
               20                  25                  30
   Ile Gly Phe Lys Glu Gln Glu Val Met Ser Gln Phe Val Asp Gln Ile
           35                  40                  45
   Asp Gly Asp Glu Tyr Ser Ile Ser Ser Gln Ala Glu Asp Val Glu Ile
       50                  55                  60
   Asp Leu Leu His Glu Phe Asp Phe Ile Pro Val Leu Ser Val Glu Leu
   65                  70                  75                  80
   Asp Pro Glu Asp Val Asp Ala Leu Glu Leu Asp Pro Ala Ile Ala Tyr
                   85                  90                  95
   Ile Glu Glu Asp Ala Glu Val Thr Thr Met Gln Thr Val Pro Trp Gly
               100                 105                 110
   Ile Asn Arg Val Gln Ala Pro Ile Ala Gln Ser Arg Gly Phe Thr Gly
           115                 120                 125
   Thr Gly Val Arg Val Ala Val Leu Asp Thr Gly Ile Ser Asn His Ala
       130                 135                 140
   Asp Leu Arg Ile Arg Gly Gly Ala Ser Phe Val Pro Gly Glu Pro Asn
   145                 150                 155                 160
   Ile Ser Asp Gly Asn Gly His Gly Thr His Val Ala Gly Thr Ile Ala
                   165                 170                 175
   Ala Leu Asn Asn Ser Ile Gly Val Leu Gly Val Ala Pro Asn Val Asp
               180                 185                 190
   Leu Tyr Gly Val Lys Val Leu Gly Ala Asp Gly Ser Gly Ser Ile Ser
           195                 200                 205
   Gly Ile Ala Gln Gly Leu Gln Trp Ala Ala Asn Asn Gly Met His Ile
       210                 215                 220
   Ala Asn Met Ser Leu Gly Ser Ser Ala Gly Ser Ala Thr Met Glu Gln
   225                 230                 235                 240
   Ala Val Asn Gln Ala Thr Ala Ser Gly Val Leu Val Val Ala Ala Ser
                   245                 250                 255
   Gly Asn Ser Gly Ala Gly Asn Val Gly Phe Pro Ala Arg Tyr Ala Asn
               260                 265                 270
   Ala Met Ala Val Gly Ala Thr Asp Gln Asn Asn Asn Arg Ala Ser Phe
           275                 280                 285
   Ser Gln Tyr Gly Ala Gly Leu Asp Ile Val Ala Pro Gly Val Gly Val
       290                 295                 300
   Gln Ser Thr Val Pro Gly Asn Gly Tyr Ala Ser Phe Asn Gly Thr Ser
   305                 310                 315                 320
   Met Ala Thr Pro His Val Ala Gly Val Ala Ala Leu Val Lys Gln Lys
                   325                 330                 335
   Asn Pro Ser Trp Ser Asn Val Gln Ile Arg Asn His Leu Lys Asn Thr
               340                 345                 350
   Ala Thr Asn Leu Gly Asn Thr Thr Gln Phe Gly Ser Gly Leu Val Asn
           355                 360                 365
   Ala Glu Ala Ala Thr Arg
       370
<210> 8
   <211> 374
   <212> PRT
   <213> Bacillus sp. DSM 14392
<400> 8
   Met Gly Lys Ile Val Ala Gly Thr Ala Leu Ile Ile Ser Val Ala Phe
   1               5                   10                  15
   Ser Ser Ser Ile Ala Gln Ala Ala Glu Glu Ala Lys Glu Lys Tyr Leu
               20                  25                  30
   Ile Gly Phe Lys Glu Gln Glu Val Met Ser Gln Phe Val Asp Gln Ile
           35                  40                  45
   Asp Gly Asp Glu Tyr Ser Ile Ser Ser Gln Ala Glu Asp Val Glu Ile
       50                  55                  60
   Asp Leu Leu His Glu Phe Asp Phe Ile Pro Val Leu Ser Val Glu Leu
   65                  70                  75                  80
   Asp Pro Glu Asp Val Asp Ala Leu Glu Leu Asp Pro Ala Ile Ala Tyr
                   85                  90                  95
   Ile Glu Glu Asp Ala Glu Val Thr Thr Met Gln Thr Val Pro Trp Gly
               100                 105                 110
   Ile Asn Arg Val Gln Ala Pro Ile Ala Gln Ser Arg Gly Phe Thr Gly
           115                 120                 125
   Thr Gly Val Arg Val Ala Val Leu Asp Thr Gly Ile Ser Asn His Ala
       130                 135                 140
   Asp Leu Arg Ile Arg Gly Gly Ala Ser Phe Val Pro Gly Glu Pro Asn
   145                 150                 155                 160
   Ile Ser Asp Gly Asn Gly His Gly Thr His Val Ala Gly Thr Ile Ala
                   165                 170                 175
   Ala Leu Asn Asn Ser Ile Gly Val Leu Gly Val Ala Pro Asn Val Asp
               180                 185                 190
   Leu Tyr Gly Val Lys Val Leu Gly Ala Ser Gly Glu Gly Ser Ile Ser
           195                 200                 205
   Gly Ile Ala Gln Gly Leu Gln Trp Ala Ala Asn Asn Gly Met His Ile
       210                 215                 220
   Ala Asn Met Ser Leu Gly Ser Ser Ala Gly Ser Ala Thr Met Glu Gln
   225                 230                 235                 240
   Ala Val Asn Gln Ala Thr Ala Ser Gly Val Leu Val Val Ala Ala Ser
                   245                 250                 255
   Gly Asn Ser Gly Ala Gly Asn Val Gly Phe Pro Ala Arg Tyr Ala Asn
               260                 265                 270
   Ala Met Ala Val Gly Ala Thr Asp Gln Asn Asn Asn Arg Ala Ser Phe
           275                 280                 285
   Ser Gln Tyr Gly Ala Gly Leu Asp Ile Val Ala Pro Gly Val Gly Val
       290                 295                 300
   Gln Ser Thr Val Pro Gly Asn Gly Tyr Ala Ser Phe Asn Gly Thr Ser
   305                 310                 315                 320
   Met Ala Thr Pro His Val Ala Gly Val Ala Ala Leu Val Lys Gln Lys
                   325                 330                 335
   Asn Pro Ser Trp Ser Asn Val Gln Ile Arg Asn His Leu Lys Asn Thr
               340                 345                 350
   Ala Thr Asn Leu Gly Asn Thr Thr Gln Phe Gly Ser Gly Leu Val Asn
           355                 360                 365
   Ala Glu Ala Ala Thr Arg
   370
<210> 9
   <211> 374
   <212> PRT
   <213> Bacillus sp. DSM 14392
<400> 9
   Met Gly Lys Ile Val Ala Gly Thr Ala Leu Ile Ile Ser Val Ala Phe
   1               5                   10                  15
   Ser Ser Ser Ile Ala Gln Ala Ala Glu Glu Ala Lys Glu Lys Tyr Leu
               20                  25                  30
   Ile Gly Phe Lys Glu Gln Glu Val Met Ser Gln Phe Val Asp Gln Ile
           35                  40                  45
   Asp Gly Asp Glu Tyr Ser Ile Ser Ser Gln Ala Glu Asp Val Glu Ile
       50                  55                  60
   Asp Leu Leu His Glu Phe Asp Phe Ile Pro Val Leu Ser Val Glu Leu
   65                  70                  75                  80
   Asp Pro Glu Asp Val Asp Ala Leu Glu Leu Asp Pro Ala Ile Ala Tyr
                   85                  90                  95
   Ile Glu Glu Asp Ala Glu Val Thr Thr Met Gln Thr Val Pro Trp Gly
               100                 105                 110
   Ile Asn Arg Val Gln Ala Pro Ile Ala Gln Ser Arg Gly Phe Thr Gly
           115                 120                 125
   Thr Gly Val Arg Val Ala Val Leu Asp Thr Gly Ile Ser Asn His Ala
       130                 135                 140
   Asp Leu Arg Ile Arg Gly Gly Ala Ser Phe Val Pro Gly Glu Pro Asn
   145                 150                 155                 160
   Ile Ser Asp Gly Asn Gly His Gly Thr His Val Ala Gly Thr Ile Ala
                   165                 170                 175
   Ala Leu Asn Asn Ser Ile Gly Val Leu Gly Val Ala Pro Asn Val Asp
               180                 185                 190
   Leu Tyr Gly Val Lys Val Leu Gly Ala Asp Gly Glu Gly Ser Ile Ser
           195                 200                 205
   Gly Ile Ala Gln Gly Leu Gln Trp Ala Ala Asn Asn Gly Met His Ile
       210                 215                 220
   Ala Asn Met Ser Leu Gly Ser Ser Ala Gly Ser Ala Thr Met Glu Gln
   225                 230                 235                 240
   Ala Val Asn Gln Ala Thr Ala Ser Gly Val Leu Val Val Ala Ala Ser
                   245                 250                 255
   Gly Asn Ser Gly Ala Gly Asn Val Gly Phe Pro Ala Arg Tyr Ala Asn
               260                 265                 270
   Ala Met Ala Val Gly Ala Thr Asp Gln Asn Asn Asn Arg Ala Ser Phe
           275                 280                 285
   Ser Gln Tyr Gly Ala Gly Leu Asp Ile Val Ala Pro Gly Val Gly Val
       290                 295                 300
   Gln Ser Thr Val Pro Gly Asn Gly Tyr Ala Ser Phe Asn Gly Thr Ser
   305                 310                 315                 320
   Met Ala Thr Pro His Val Ala Gly Val Ala Ala Leu Val Lys Gln Lys
                   325                 330                 335
   Asn Pro Ser Trp Ser Asn Val Gln Ile Arg Asn His Leu Lys Asn Thr
               340                 345                 350
   Ala Thr Asn Leu Gly Asn Thr Thr Gln Phe Gly Ser Gly Leu Val Asn
           355                 360                 365
   Ala Glu Ala Ala Thr Arg
       370